# EUROPÄISCHE PATENTANMELDUNG

(11) **EP 2 204 161 A2**
(43) Veröffentlichungstag der Anmeldung: **07.07.2010**
(21) Anmeldenummer: 09169403.4
(22) Anmeldetag: 03.09.2009
(51) Int. Cl.: A61K 8/35, A61K 8/92, A61Q 5/00, A61Q 19/00

(54) **Kosmetische Zusammensetzung enthaltend ein Öl aus den Früchten, insbesondere den Kernen von Pflanzen der Ordnung Rosales**

(30) Priorität: 06.09.2008 DE 102008046178
(71) Anmelder: Henkel AG & Co. KGaA, 40589 Düsseldorf (DE)
(72) Erfinder: Battermann, Marlene, 21271, Asendorf (DE); Gröning, Melanie, 81379 München (DE); Goddinger, Dieter, 25336, Klein Nordende (DE); Hippe, Thomas, 25482, Appen (DE)

(57) **Zusammenfassung**

Die Erfindung betrifft Zusammensetzungen enthaltend mindestens ein Öl aus der Frucht, insbesondere den Kernen einer Pflanze der Ordnung Rosales sowie eine das UV - Licht absorbierende Verbindung und eine filmbildende Verbindung.

## Beschreibung

Die Erfindung betrifft kosmetische Mittel enthaltend ein Öl aus den Früchten, insbesondere den Kernen von Pflanzen der Ordnung Rosales in einem kosmetischen Träger und weitere Wirkstoffe. Es besteht ein Bedarf nach Wirkstoffen bzw. Wirkstoffkombinationen für kosmetische Mittel mit guten pflegenden Eigenschaften und guter biologischer Abbaubarkeit. Insbesondere in farbstoff- und/oder elektrolythaltigen Formulierungen besteht Bedarf an zusätzlichen pflegenden Wirkstoffen, die sich problemlos in bekannte Formulierungen einarbeiten lassen.

Gesucht sind als pflegende Wirkstoffe daher insbesondere bereits in kosmetischen Mitteln seit langem verwendete Inhaltsstoffe, welche sich durch eine besonders gute Verträglichkeit auszeichnen. Gleichzeitig dürfen sich jedoch das Wirkungsspektrum und das Eigenschaftsprofil der kosmetischen Zusammensetzung durch die Verwendung der milden und besonders gut verträglichen Inhaltsstoffe nicht gegenüber den herkömmlichen Zusammensetzungen nachteilig verändern. Dies wird weiterhin dadurch erschwert, dass in den erfindungsgemäßen Zusammensetzungen bewusst nur so wenige Inhaltsstoffe wie unbedingt notwendig verwendet werden.

Überraschenderweise wurde nun gefunden, dass ein Öl, welches aus den Früchten, insbesondere aus den Kernen von Pflanzen der Ordnung Rosales gewonnen wird, sich hervorragend dazu eignet in kosmetischen Zusammensetzungen verwendet zu werden. Die überraschenden Wirkungen zeigen sich insbesondere dann, wenn das Öl aus den Früchten, insbesondere den Kernen von Pflanzen der Ordnung der Rosales gemeinsam mit mindestens einem UV - Filter und mindestens einer weiteren Substanz mit filmbildenden Eigenschaften in einem wäßrigen Träger verwendet wird.

Die Ordnung Rosales ist in 24 Familien untergliedert. Allen Familien der Ordnung Rosales ist jedoch gemein, daß sie sich nie an Parasiten und nie an eine aquatische Lebensweise angepasst haben. Manchmal wird die Zuordnung daher auf die Familie der Rosaceae beschränkt. Die Ordnung Rosales wiederum ist eine von 18 Ordnungen innerhalb der Unterklasse der Rosidae. Ein erster Gegenstand der vorliegenden Erfindung ist daher eine Zusammensetzung zur Behandlung der Haut und von keratinischen Fasern, enthaltend
a) mindestens ein oder mehrere Öle, gewonnen aus Früchten, insbesondere den Kernen mindestens einer Pflanze aus der Ordnung Rosales,
b) mindestens ein UV - Filter,
c) mindestens eine Verbindung mit filmbildenden Eigenschaften und ein kosmetischer Träger.

Der erfindungsgemäße Wirkstoffkomplex trägt zur Verbesserung der Verträglichkeit der kosmetischen Zusammensetzungen für Haut und Haar bei, beeinflusst die innere und äußere Struktur von Haut und Haar positiv, erhöht die Elastizität der Haut, erhöht die Reißkräfte des nassen wie des trockenen Haares, trägt zur Restrukturierung des Haares bei, fördert und erhöht die Glätte von Haut und Haar und erzeugt einen angenehmen Griff und ein angenehmes Gefühl auf Haut und Haar im jeweils nassen wie trockenen Zustand. Weiterhin wird insbesondere eine Erhöhung der Echtheiten der gefärbter und / oder blondierten keratinischen Faser, beispielsweise Waschechtheit, Farbechtheit, Auswaschbeständigkeit, UV - Beständigkeit, Schweißbeständigkeit, erzielt. Eine ganz besonders positive Eigenschaft ist, dass in Haarbehandlungsmitteln oder Stylingmitteln das Volumen und die Festigkeit von keratinischen Fasern, sowie die Haltbarkeit einer Dauerwelle deutlich verbessert werden. In Stylingmitteln wirkt der erfindungsgemäße Komplex zusätzlich als Weichmacher für die aus polymeren Filmbildnern gebildeten Filme. Weiterhin zeichnen sich die erfindungsgemäßen Zusammensetzungen enthaltend den erfindungsgemäßen Wirkstoffkomplex durch einen deutlich verbesserten Zustand der Haut und des Haares in Bezug auf den Feuchtehaushalt der trockenen Haut und des trockenen Haares aus. Schließlich wurde überraschender Weise festgestellt, dass die erfindungsgemäßen Zusammensetzungen zu einer deutlich verzögerten Wiederanschmutzung von Haut und Haar führen. Die Benetzung von Haut und Haar mit Verunreinigungen und Wasser wird deutlich verzögert. Dieser Lotuseffekt wiederum trägt entscheidend mit dazu bei, dass gerade trockene und empfindliche Haut sowie trockenes und sprödes Haar im Vergleich zu üblichen kosmetischen Mitteln gemäß dem Stand der Technik ohne die erfindungsgemäße Zusammensetzung nicht so häufig behandelt werden müssen. Hierdurch werden insbesondere die empfindliche und trockene Haut sowie trockenes und strapaziertes Haar deutlich weniger geschädigt. In höchstem Maße überraschend zeigen die erfindungsgemäßen Zusammensetzungen eine signifikante und deutliche Erhöhung der UV - Beständigkeit der damit behandelten Haut und der damit behandelten keratinischen Fasern.

Die Inhaltsstoffe a), b) und c) werden nachfolgend detailliert beschrieben. Soweit nachstehend vom Wirkstoffkomplex (A) gesprochen wird, bezieht sich diese Aussage auf die in den erfindungsgemäßen Mitteln zwingend enthaltenen Inhaltsstoffe a) bis c).

Haarbehandlungsmittel im Sinne der vorliegenden Erfindung sind beispielsweise Haarfärbemittel, Blondiermittel, Haarshampoos, Haarkonditionierer, konditionierende Shampoos, Haarsprays, Haarspülungen, Haarkuren, Haarpackungen, Haar-Tonics, Dauerwell-Fixierlösungen, Haarfärbeshampoos, Haarfärbemittel, Haarfestiger, Haarlegemittel, Haarstyling-Zubereitungen, Fönwell-Lotionen, Schaumfestiger, Haargele, Haarwachse oder deren Kombinationen. Im Hinblick auf die Tatsache, daß der Verbraucher, insbesondere jedoch Männer oft die Anwendung mehrerer unterschiedlicher Mittel und/oder mehrere Anwendungsschritte scheuen, sind erfindungsgemäße Mittel bevorzugt solche Mittel, welche der Verbraucher, insbesondere der Mann ohnehin anwendet. Bevorzugte erfindungsgemäße Mittel sind daher Shampoos, Konditioniermittel oder Haar-Tonics. Unter Kämmbarkeit versteht sich erfindungsgemäß sowohl die Kämmbarkeit der nassen Faser, als auch die Kämmbarkeit der trockenen Faser. Als Maß für die Kämmbarkeit dient die aufgewendete Kämmarbeit oder die aufgewendete Kraft während des Kämmvorganges eines Faserkollektivs. Die Meßparameter können durch den Fachmann sensorisch beurteilt oder durch Messeinrichtungen quantifiziert werden.

Als Griff definiert sich die Taktilität eines Faserkollektivs, wobei der Fachmann sensorisch die Parameter Fülle und Geschmeidigkeit des Kollektivs fühlt und bewertet.

Unter Formgebung wird die Fähigkeit verstanden, einem Kollektiv zuvor behandelter keratinhaltiger Fasern, insbesondere menschlicher Haare, eine Formänderung zu verleihen. In der Haarkosmetik wird auch von Frisierbarkeit gesprochen.

Unter der Aufrechterhaltung des natürlichen Wachstum keratinischer Fasern wird verstanden, dass die Einflüsse auf das natürliche Haarwachstum durch haarkosmetische Behandlungen wie zuvor dargestellt, insbesondere durch oxidative Haarbehandlungen ausgeglichen werden und keine oder allenfalls geringe Auswirkungen auf das natürliche Wachstum der keratinischen Fasern in Bezug auf das Dickenwachstum, das Längenwachstum und/oder in Bezug auf die Haarfülle vorhanden sind. Das Dickenwachstum, das Längenwachstum oder die Haarfülle kann dabei sowohl subjektiv als auch objektiv oder in verschiedenen Testmodellen bestimmt werden. Unter einer oxidativen Haarbehandlung wird erfindungsgemäß die Einwirkung eines oxidativen kosmetischen Mittels, enthaltend in einem kosmetischen Träger mindestens ein Oxidationsmittel, auf Haar definiert.

Als kosmetische Träger eignen sich erfindungsgemäß besonders O/W - , W/O - und W/O/W - Emulsionen in Form von Cremes oder Gelen oder auch tensidhaltige schäumende Lösungen, wie beispielsweise Shampoos, Schaumaerosole oder andere Zubereitungen, die insbesondere für die Anwendung auf dem Haar geeignet sind. Es ist aber auch denkbar, die Inhaltsstoffe in eine pulverförmige oder auch tablettenförmige Formulierung zu integrieren, welche vor der Anwendung in Wasser gelöst wird. Die kosmetischen Träger können insbesondere wässrig oder wässrigalkoholisch sein.

Ein wässriger kosmetischer Träger enthält mindestens 50 Gew.-% Wasser. Bevorzugt sind solche wässrigen kosmetischen Träger, welche mindestens 70 Gew.% Wasser, besonders bevorzugt 80 Gew.% Wasser enthalten.

Unter wässrig-alkoholischen kosmetischen Trägern sind im Sinne der vorliegenden Erfindung wässrige Lösungen enthaltend 3 bis 70 Gew.-% eines C₁-C₆-Alkohols, insbesondere Methanol, Ethanol bzw. Propanol, Isopropanol, Butanol, Isobutanol, tert.-Butanol, n-Pentanol, iso-Pentanole, n-Hexanol, iso-Hexanole, Glykol, Glycerin, 1,2-Pentandiol, 1,5-Pentandiol, 1,2-Hexandiol oder 1,6-Hexandiol zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Als Inhaltstoff a) enthalten die erfindungsgemäßen Mittel mindestens ein Öl, welches aus den Früchten, insbesondere den Kernen einer Pflanze der Ordnung Rosales gewonnen wird.

Innerhalb der Ordnung der Rosales wird das Öl, welches aus den Früchten, insbesondere den Kernen der Rosales gewonnen wird und in kosmetischen Zusammensetzungen, insbesondere in Zusammensetzungen zur Behandlung von keratinischen Fasern verwendet wird, bevorzugt aus den Früchten, insbesondere den Kernen einer der Familien Rosaceae, Crassulaceae oder Saxifragaceae gewonnen.

Ein bevorzugtes Öl aus den Früchten, insbesondere den Kernen der Rosales wird aus der Familie der Rosaceae erhalten. Das Öl, welches aus den Früchten, insbesondere den Kernen der Rosoideae gewonnen wird, ist besonders bevorzugt.

Höchst bevorzugt ist das Öl, welches aus den Früchten, insbesondere den Kernen der Rosoideae gewonnen wird.

Am bevorzugtesten ist jedoch das Öl, welches aus den Früchten, insbesondere den Kernen von Rosen, den Hagebutten, gewonnen wird. Bei den Rosen kann es sich dabei sowohl um Wildformen als auch um Züchtungen handeln. Rosen zählen zur Gattung Rosa.

Die erfindungsgemäßen Öle werden durch die dem Fachmann bekannten und üblichen Verfahren gewonnen.

Besonders bevorzugt und schonend werden die Öle aus den Früchten, insbesondere den Kernen der Rosales jedoch nach den sogenannten kalten Pressverfahren erhalten. Selbstverständlich kann die Kaltpressung mit den ganzen Früchten oder nur den Kernen, den Samen durchgeführt werden.

Erfindungsgemäß am bevorzugtesten ist das Öl, welches unter die INCI - Bezeichnung Rosa Canina im Handel erhältlich ist.

Das Öl aus den Früchten, insbesondere den Kernen der Rosales ist in den erfindungsgemäßen Zusammensetzungen in Mengen von 0,01 bis 20 Gew.%, bevorzugt in Mengen von 0,01 bis 10 Gew.% und ganz besonders bevorzugt in Mengen von 0,05 bis 7,5 Gew.% enthalten. Die allerbesten Ergebnisse werden dabei mit Mengen von 0,1 bis 5 Gew.% jeweils bezogen auf die Gesamtzusammensetzung des jeweiligen Mittels erhalten.

UV - Filter sind die zweite zwingende Komponente, der Inhaltsstoff b), der erfindungsgemäßen Wirkstoffkombination (A). Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Beispiele für erfindungsgemäß verwendbar UV-Filter sind 4-Amino-benzoesäure, N,N,N-Trimethyl-4-(2-oxoborn-3-ylidenmethyl)anilin-methylsulfat, 3,3,5-Trimethyl-cyclohexylsalicylat (Homosalate), 2-Hydroxy-4-methoxy-benzophenon (Benzophenone-3; Uvinul^{®}M 40, Uvasorb^{®}MET, Neo Heliopan^{®}BB, Eusolex^{®}4360), 2-Phenylbenzimidazol-5-sulfonsäure und deren Kalium-, Natrium- und Triethanolaminsalze (Phenylbenzimidazole sulfonic acid; Parsol^{®}HS; Neo Heliopan^{®}Hydro), 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion (Butyl methoxydibenzoylmethane; Parsol^{®}1789, Eusolex^{®}9020), α-(2-Oxoborn-3-yliden)-toluol-4-sulfonsäure und deren Salze, ethoxylierte 4-Aminobenzoesäure-ethylester (PEG-25 PABA; Uvinul^{®}P 25), 4-Dimethylaminobenzoesäure-2-ethylhexylester (Octyl Dimethyl PABA; Uvasorb^{®}DMO, Escalol^{®}507, Eusolex^{®}6007), Salicylsäure-2-ethylhexylester (Octyl Salicylat; Escalol^{®}587, Neo Heliopan^{®}OS, Uvinul^{®}018), 4-Methoxyzimtsäure-isopentylester (Isoamyl p-Methoxycinnamate; Neo Heliopan^{®}E 1000), 4-Methoxyzimtsäure-2-ethylhexyl-ester (Octyl Methoxycinnamate; Parsol^{®}MCX, Escalol^{®}557, Neo Heliopan^{®}AV), 2-Hydroxy-4-methoxybenzophenon-5-sulfonsäure und deren Natriumsalz (Benzophenone-4; Uvinul^{®}MS 40; Uvasorb^{®}S 5), 3-(4'-Methylbenzyliden)-D,L-Campher (4-Methylbenzylidene camphor; Parsol^{®}5000, Eusolex^{®}6300), 3-Benzyliden-campher (3-Benzylidene camphor), 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamids, 2,4-Dihydroxybenzophenon (Benzophenone-1; Uvasorb^{®}20 H, Uvinul^{®}400), 1,1'-Diphenylacrylonitrilsäure-2-ethylhexyl-ester (Octocrylene; Eusolex^{®}OCR, Neo Heliopan^{®}Type 303, Uvinul^{®}N 539 SG), o-Aminobenzoesäure-menthylester (Menthyl Anthranilate; Neo Heliopan^{®}MA), 2,2',4,4'-Tetrahydroxybenzophenon (Benzophenone-2; Uvinul^{®}D-50), 2,2'-Dihydroxy-4,4'-dimethoxybenzophenon (Benzophenone-6), 2,2`-Dihydroxy-4,4`-dimethoxybenzo-phenon-5-natriumsulfonat und 2-Cyano-3,3-diphenylacrylsäure-2'-ethylhexylester. Bevorzugt sind 4-Amino-benzoesäure, N,N,N-Trimethyl-4-(2-oxoborn-3-ylidenmethyl)anilin-methylsulfat, 3,3,5-Trimethyl-cyclohexylsalicylat, 2-Hydroxy-4-methoxy-benzophenon, 2-Phenylbenzimidazol-5-sulfonsäure und deren Kalium-, Natrium- und Triethanolaminsalze, 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion, α-(2-Oxoborn-3-yliden)-toluol-4-sulfonsäure und deren Salze, ethoxylierte 4-Aminobenzoesäure-ethylester, 4-Dimethylaminobenzoesäure-2-ethylhexylester, Salicylsäure-2-ethylhexylester, 4-Methoxyzimtsäure-isopentylester, 4-Methoxyzimtsäure-2-ethylhexyl-ester, 2-Hydroxy-4-methoxybenzophenon-5-sulfonsäure und deren Natriumsalz, 3-(4'-Methylbenzyliden)-D,L-Campher, 3-Benzyliden-campher, 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamid. Erfindungsgemäß ganz besonders bevorzugt sind 2-Hydroxy-4-methoxy-benzophenon, 2-Phenylbenzimidazol-5-sulfonsäure und deren Kalium-, Natrium- und Triethanolaminsalze, 1-(4-tert.-Butylphenyl)-3-(4-methoxyphenyl)-propan-1,3-dion, 4-Methoxyzimtsäure-2-ethylhexyl-ester und 3-(4'-Methylbenzyliden)-D,L-Campher.

Bevorzugt sind solche UV-Filter, deren molarer Extinktionskoeffizient am Absorptionsmaximum oberhalb von 15 000, insbesondere oberhalb von 20000, liegt.

Weiterhin wurde gefunden, daß bei strukturell ähnlichen UV-Filtern in vielen Fällen die wasserunlösliche Verbindung im Rahmen der erfindungsgemäßen Lehre die höhere Wirkung gegenüber solchen wasserlöslichen Verbindungen aufweist, die sich von ihr durch eine oder mehrere zusätzlich ionische Gruppen unterscheiden. Als wasserunlöslich sind im Rahmen der Erfindung solche UV-Filter zu verstehen, die sich bei 20 °C zu nicht mehr als 1 Gew.-%, insbesondere zu nicht mehr als 0,1 Gew.-%, in Wasser lösen. Weiterhin sollten diese Verbindungen in üblichen kosmetischen Ölkomponenten bei Raumtemperatur zu mindestens 0,1, insbesondere zu mindestens 1 Gew.-% löslich sein). Die Verwendung wasserunlöslicher UV-Filter kann daher erfindungsgemäß bevorzugt sein.

Erfindungsgemäß können UV-Filter bevorzugt sein, die eine kationische Gruppe, insbesondere eine quartäre Ammoniumgruppe, aufweisen.

Zwei bevorzugte UV-Filter mit kationischen Gruppen sind die als Handelsprodukte erhältlichen Verbindungen Zimtsäureamidopropyl-trimethylammoniumchlorid (Incroquat^{®}UV-283) und Dodecyl-dimethylaminobenzamidopropyl-dimethylammoniumtosylat (Escalol^{®} HP 610). Erfindungsgemäß besonders bevorzugt sind als UV - Filter diejenigen, welche zur Gruppe der substituierten Benzophenone, p-Aminobenzoesäureester, Diphenylacrylsäureester, Zimtsäureester, Zimtsäureamidopropyl-trialkylammoniumchloride, Salicylsäureester, Octocrylene, Benzimidazole, Dodecyl-dimethylaminobenzamidopropyl-dimethylammoniumtosylate und o-Aminobenzoesäureester zugehörig sind. Als höchst bevorzugte UV - Filter sind zu nennen: Benzophenone-4, Octocrylen, Zimtsäureamidopropyl-trimethylammoniumchlorid und Dodecyl-dimethylaminobenzamidopropyl-dimethylammoniumtosylat.

Die UV-Filter (I) sind in den erfindungsgemäß verwendeten Mitteln üblicherweise in Mengen 0,1-5 Gew.-%, bezogen auf das gesamte Mittel, enthalten. Mengen von 0,4-2,5 Gew.-% sind bevorzugt. Der erfindungsgemäße Inhaltsstoff c), eine Verbindung, welche filmbildende Eigenschaften aufweist, ist die dritte zwingende Komponente. Unter dem Begriff "filmbildend" ist zu verstehen, daß auf einer Oberfläche wie der Haut oder einer keratinischen Faser über eine größere Fläche hinweg ein überwiegend geschlossener Film ergibt. Selbstverständlich kann dieser Film durchbrochen sein, beispielsweise durch Fehlstellen, wenn die Oberfläche zu rauh und zu ungleichmäßig ist. Weiterhin wird erfindungsgemäß unter "filmbildend" auch verstanden, wenn der Oberfläche der Haut oder von keratinischen Fasern Substanzen anhaften, welche durch ihre hydrophilen Eigenschaften einen wäßrigen Film, eine ausgeprägte Hydrathülle zu bilden. Die Erfinder haben festgestellt, daß die vorliegende Erfindung mit 3 unterschiedlichen filmbildenden Substanzklassen besonders gute Ergebnisse zeigt. Die erste Gruppe filmbildender Substanzen gemäß der vorliegenden Erfindung sind c1) Polymere, insbesondere kationische Polymere, amphotere Polymere und anionische Polymere. Die Polymere werden ausführlich an anderer Stelle beschrieben. An dieser Stelle wird auf die besonders geeigneten Polymere eingegangen.

Beispielhaft für die erfindungsgemäßen besonders geeigneten Polymere als Inhaltsstoff c1) stehen Verbindungen mit der INCII - Bezeichnung: Polyquaternium-2, Polyquaternium-4, Polyquaternium-6, Polyquaternium-7, Polyquaternium-10, Polyquaternium-11, Polyquaternium-16, Polyquaternium-22, Polyquaternium-28, Polyquaternium-32, Polyquaternium-37, Polyquaternium-39, Polyquaternium-44, Polyquaternium-46 und Polyquaternium-47.

Die zweite Gruppe c2) für filmbildende Substanzen im Sinne der vorliegenden Erfindung sind Glycerine, Glykole wie Propylenglykol, Polyglykole und Propylenglykole mit einem Ethoxilierungsgrad von 1 Mol Ethylenoxid bis hin zu 100 Mol Ethylenoxid, bevorzugt von 1 Mol bis zu 50 Mol, besonders bevorzugt von 1 Mol bis 20 Mol Ethylenoxid, höchst bevorzugt von 1 Mol bis zu 10 Mol Ethylenoxid, wobei insbesondere Glykol, Diglykol, Triglykol, Propylenglykol, Butylenglykol, Glycerin, Diglycerin, Triglycerin, Polyglycerin sowie deren Ethoxilate mit Ethoxilierungsgraden von 1 Mol Ethylenoxid bis hin zu 100 Mol Ethylenoxid, bevorzugt von 1 Mol bis zu 50 Mol, besonders bevorzugt von 1 Mol bis 20 Mol Ethylenoxid, höchst bevorzugt von 1 Mol bis zu 10 Mol Ethylenoxid, bevorzugt sind.

Filmbildende Substanzen c3) im Sinne der vorliegenden Erfindung sind Vitamin B₅ und dessen Derivate, beispielsweise Pantothensäure, Panthenol und Pantolacton. Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate. Unter Panthothensäure sind erfindungsgemäß auch die physiologisch verträglichen Salze, wie die Natrium-, Kalium-, Magnesium- oder Calciumsalze zu verstehen. Weiterhin können die Ester der Panthothensäure verwendet werden.

Selbstverständlich ist es erfindungsgemäß möglich mehrere der zuvor genannten filmbildenden Substanzen zu verwenden. In diesem Falle ist es höchst bevorzugt, wenn die filmbildenden Substanzen aus mindestens zwei unterschiedlichen Kategorien der filmbildenden Substanzen gewählt werden. Höchst bevorzugt ist die Auswahl aus der Gruppe c1) und c3). Weiterhin können im Falle, dass mehrere der filmbildenden Inhaltsstoffe verwendet werden selbstverständlich auch mehrere dieser Inhaltsstoffe aus einer Kategorie gewählt werden, sofern mindestens zwei der Inhaltsstoffe unterschiedlichen Kategorien angehören. Hierbei ist bevorzugt, wenn mindestens zwei Polymere, insbesondere mindestens zwei kationische Polymere enthalten sind. Die filmbildenden Substanzen c) sind in den erfindungsgemäßen Zusammensetzungen in einer Menge von 0,01 bis 10 Gew.%, bevorzugt in einer Menge von 0,01 bis 7,5 Gew.%, insbesondere in einer Menge von 0,05 bis 7,0 Gew.% jeweils bezogen auf die gesamte Zusammensetzung enthalten.

Die bevorzugten erfindungsgemäßen Zusammensetzungen enthalten neben einem kosmetischen Träger die folgenden Inhaltsstoffe:
a) mindestens ein Öl, welches aus den Früchten, insbesondere den Kernen der Rosoideae gewonnen wird,
b) mindestens einen UV - Filter, welcher zur Gruppe der substituierten Benzophenone, p-Aminobenzoesäureester, Diphenylacrylsäureester, Zimtsäureester, Zimtsäureamidopropyl-trialkylammoniumchloride, Salicylsäureester, Octocrylene, Benzimidazole, Dodecyl-dimethylaminobenzamidopropyl-dimethylammoniumtosylate und/oder o-Aminobenzoesäureester zugehörig ist,
c) mindestens eine filmbildende Verbindung ausgewählt aus c1) Polyquaternium-2, Polyquaternium-4, Polyquaternium-6, Polyquaternium-7, Polyquaternium-10, Polyquaternium-11, Polyquaternium-16, Polyquaternium-22, Polyquaternium-28, Polyquaternium-32, Polyquaternium-37, Polyquaternium-39, Polyquaternium-44, Polyquaternium-46, Polyquaternium-47, c2) Glycerine, Glykole wie Propylenglykol, Polyglykole und Propylenglykole mit einem Ethoxilierungsgrad von 1 Mol Ethylenoxid bis hin zu 100 Mol Ethylenoxid, bevorzugt von 1 Mol bis zu 50 Mol, besonders bevorzugt von 1 Mol bis 20 Mol Ethylenoxid, höchst bevorzugt von 1 Mol bis zu 10 Mol Ethylenoxid, wobei insbesondere Glykol, Diglykol, Triglykol, Propylenglykol, Butylenglykol, Glycerin, Diglycerin, Triglycerin, Polyglycerin sowie deren Ethoxilate mit Ethoxilierungsgraden von 1 Mol Ethylenoxid bis hin zu 100 Mol Ethylenoxid, bevorzugt von 1 Mol bis zu 50 Mol, besonders bevorzugt von 1 Mol bis 20 Mol Ethylenoxid, höchst bevorzugt von 1 Mol bis zu 10 Mol Ethylenoxid, c3) Vitamin B₅ und dessen Derivate, beispielsweise Pantothensäure, Panthenol und Pantolacton.

Höchst bevorzugte erfindungsgemäße Zusammensetzungen enthalten neben einem kosmetischen Träger beispielsweise:
a) mindestens ein Öl, welches aus den Früchten, insbesondere den Kernen von Rosen, den Hagebutten, gewonnen wird,
b) mindestens einen UV - Filter ausgewählt aus Benzophenone-4, Octocrylen, Zimtsäureamidopropyl-trimethylammoniumchlorid und Dodecyl-dimethylaminobenzamidopropyl-dimethylammoniumtosylat und
c) mindestens eine filmbildende Verbindung c1) ausgewählt aus Polyquaternium-2, Polyquaternium-4, Polyquaternium-6, Polyquaternium-7, Polyquaternium-10, Polyquaternium-11, Polyquaternium-16, Polyquaternium-22, Polyquaternium-28, Polyquaternium-32, Polyquaternium-37, Polyquaternium-39, Polyquaternium-44, Polyquaternium-46, Polyquaternium-47 und mindestens eine filmbildende Verbindung c3) ausgewählt aus Vitamin B₅ und dessen Derivaten, insbesondere Panthenol und / oder Pantolacton.

Als weiterer besonders bevorzugter Inhaltsstoff e) werden Esteröle verwendet. Insbesondere führen sie zu einer deutlich erhöhten Auswaschbeständigkeit gefärbter keratinischer Fasern und zu einem erheblich verbesserten UV - Schutz von Haut und Haar. Gleichzeitig zeigen die behandelten keratinischen Fasern einen angenehmen und glatten Griff sowohl der nassen als auch der trockenen Fasern. Die Verteilung der Wirkstoffe wird durch die Esteröle in hervorragender Weise gleichmäßig beeinflußt, so daß die Wirkstoffe gleichmäßig auf der Oberfläche von Haut und Haar verteilt sind.

Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).

Selbstverständlich können die Esteröle auch mit Etyhlenoxid, Propylenoxid oder Mischungen aus Ethylenoxid und Propylenoxid alkoxiliert sein. Die Alkoxylierung kann dabei sowohl am Fettalkoholpart als auch am Fettsäurepart als auch an beiden Teilen der Esteröle zu finden sein. Bevorzugt ist erfindungsgemäß jedoch, wenn zunächst der Fettalkohol alkoxyliert wurde und anschließend mit Fettsäure verestert wurde. In der Formel (D4-II) sind allgemein diese Verbindungen dargestellt.

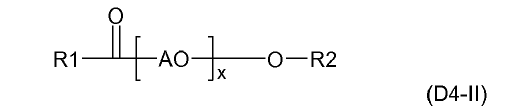

R1 steht hierbei für einen gesättigten oder ungesättigten, verzweigten oder unverzweigten, cyclischen gesättigten cyclischen ungesättigten Acylrest mit 6 bis 30 Kohlenstoffatomen, AO steht für Ethylenoxid, Propylenoxid oder Butylenoxid,

X steht für eine Zahl zwischen 1 und 200, vorzugsweise 1 und 100, besonders bevorzugt zwischen 1 und 50, ganz besonders bevorzugt zwischen 1 und 20, höchst bevorzugt zwischen 1 und 10 und am bevorzugtesten zwischen 1 und 5,

R2 steht für einen gesättigten oder ungesättigten, verzweigten oder unverzweigten, cyclischen gesättigten cyclischen ungesättigten Alkyl-, Alkenyl-, Alkinyl-, Phenyl- oder Benzylrest mit 6 bis 30 Kohlenstoffatomen. Ein erfindungsgemäß besonders bevorzugtes Esteröl ist beispielsweise unter der INCI - Bezeichnung PPG-3 Benzyl Ether Myristate erhältlich.

Weiterhin sind unter Esterölen zu verstehen:
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat, sowie
- symmetrische, unsymmetrische oder cyclische Ester der Kohlensäure mit Fettalkoholen, beispielsweise Glycerincarbonat oder Dicaprylylcarbonat (Cetiol^{®} CC),
- Trifettsäureester von gesättigten und/oder ungesättigten linearen und/oder verzweigten Fettsäuren mit Glycerin,
- Fettsäurepartialglyceride, das sind Monoglyceride, Diglyceride und deren technische Gemische. Bei der Verwendung technischer Produkte können herstellungsbedingt noch geringe Mengen Triglyceride enthalten sein. Vorzugsweise werden Ölsäuremonoglyceride eingesetzt.

Die Esteröle werden in den erfindungsgemäßen Mitteln in einer Menge von 0,01 bis 20 Gew.%, bevorzugt 0,01 bis 10,0 Gew.%, besonders bevorzugt 0,01 bis 7,5 Gew.%, höchst bevorzugt von 0,1 bis 5,0 Gew.% verwendet.

Erfindungsgemäß bevorzugt enthalten die erfindungsgemäßen Mittel weiterhin mindestens eine quartäre Imidazolinverbindung, d.h. eine Verbindung, die einen positiv geladenen Imidazolinring aufweist. Die im folgenden dargestellte Formel la zeigt die Struktur dieser Verbindungen.

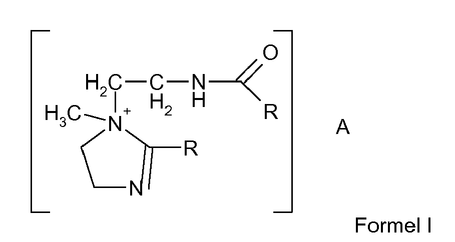

Die Reste R stehen unabhängig voneinander jeweils für einen gesättigten oder ungesättigten, linearen oder verzweigten Kohlenwasserstoffrest mit einer Kettenlänge von 8 bis 30 Kohlenstoffatomen. Die bevorzugten Verbindungen der Formel la enthalten für R jeweils den gleichen Kohlenwasserstoffrest. Die Kettenlänge der Reste R beträgt mindestens 12 Kohlenstoffatome. Bevorzugt sind Verbindungen mit einer Kettenlänge von mindestens 16 Kohlenstoffatomen, besonders bevorzugt mit mindestens 18 Kohlenstoffatomen und höchst bevorzugt mit mindestens 20 Kohlenstoffatomen. A bedeutet ein physiologisch verträgliches Anion. Erfindungsgemäß umfasst sind als anionisches Gegenion Halogenide, beispielsweise Fluorid, Chlorid oder Bromid, Alkylsulfate, wie Methosulfat oder Ethosulfat, Phosphate, Citrat, Tartrat, Maleat oder Fumarat. Diese Anionen stehen erfindungsgemäß in allen Fällen auch für die im folgenden beschriebenen Kationen als deren Gegenion.

Besonders erfindungsgemäße Beispiele sind beispielsweise unter den INCII - Bezeichnungen Quaternium-27, Quaternium-72, Quaternium-83 und Quaternium-91 bekannt

Die Imidazoline der Formel I sind in den erfindungsgemäßen Zusammensetzungen in Mengen von 0,01 bis 20 Gew.%, bevorzugt in Mengen von 0,01 bis 10 Gew.% und ganz besonders bevorzugt in Mengen von 0,1 bis 7,5 Gew.% enthalten. Die allerbesten Ergebnisse werden dabei mit Mengen von 0,1 bis 5 Gew.% jeweils bezogen auf die Gesamtzusammensetzung des jeweiligen Mittels erhalten.

Weiterhin können die folgenden kationischen Tenside gemäß der Formel (Tkat-2) entweder als alleinige kationische Tenside oder ganz besonders bevorzugt als weitere kationische Tenside zusätzlich zu den zuvor genannten Imidazolinen gemäß der Formeln I verwendet werden. RCO-X-N⁺R¹R²R³R⁴ A⁻ (Tkat-2)

R steht hierin für einen substituierten oder unsubstituierten, verzweigten oder geradkettigen Alkyl-oder Alkenylrest mit 11 bis 35 Kohlenstoffatomen in der Kette,

X steht für - O - oder - NR⁵ -,

R¹ steht für eine Alkylengruppe mit 2 bis 6 C - Atomen, welche nicht substituiert oder substituiert sein kann, wobei im Falle einer Substitution die Substitution mit einer -OH - oder -NH- Gruppe bevorzugt ist,

R², R³ und R⁴ jeweils unabhängig voneinander stehen für eine Alkyl oder Hydroxyalkylgruppe mit 1 bis zu 6 C - Atomen in der Kette, wobei die Kette geradlinig oder verzweigt sein kann. Beispiele für erfindungsgemäße Reste sind Methyl, Ethyl, Propyl, iso-Propyl, Butyl, iso-Butyl, tert-Butyl, Pentyl, iso-Pentyl, neo-Pentyl, Hexyl, iso-Hexyl, Hydroxyalkyl, Dihydroxyalkyl, Hydroxyethyl, Hydroxypropyl, Dihydroxypropyl, Hydroxybutyl, Dihydroxybutyl, Trihydroxybutyl, Trihydroxypropyl, Dihydroxyethyl, R5 steht für Wasserstoff oder einen C1 bis C6 geradkettigen oder verzweigten, Alkyl- oder Alkenylrest, welcher auch durch eine Hydroxygruppe substituiert sein kann, besonders Methyl, Ethyl, Propyl, iso-Propyl, Butyl, iso-Butyl, tert-Butyl, Pentyl, iso-Pentyl, neo-Pentyl, Hexyl, iso-Hexyl, Hydroxyethyl, Hydroxypropyl, Dihydroxypropyl, Hydroxybutyl, Dihydroxybutyl, Trihydroxybutyl, Trihydroxypropyl, Dihydroxyethyl und

Innerhalb dieser Strukturklasse werden bevorzugt die Verbindungen einer der folgenden Strukturen verwendet:

CH₃(CH₂)₂₀CONH(CH₂)₃ - N⁺(CH₃)₂-CH₂CH₃ A⁻ (Tkat-3)

CH₃(CH₂)₂₀CONH(CH₂)₃ - N⁺(CH₃)₂-CH₂(CHOH)CH₂OH A⁻ (Tkat-4)

CH₃(CH2)₂₀COOCH₂CHOHCH₂ - N⁺(CH₃)₃ A⁻ (Tkat-5)

CH₃(CH₂)₂₀CONH(CH₂)₃ - N⁺(CH₃)₂-CH₂CH₂OH A⁻ (Tkat-6)

Beispiele für derartige Handelsprodukte sind Schercoquat BAS, Lexquat AMG-BEO, Akypoquat 131 oder Incroquat Behenyl HE.

Die kationischen Tenside der Formel (Tkat-2) sind in den erfindungsgemäßen Zusammensetzungen in Mengen von 0,01 bis 20 Gew.%, bevorzugt in Mengen von 0,01 bis 10 Gew.% und ganz besonders bevorzugt in Mengen von 0,1 bis 7,5 Gew.% enthalten. Die allerbesten Ergebnisse werden dabei mit Mengen von 0,1 bis 5 Gew.% jeweils bezogen auf die Gesamtzusammensetzung des jeweiligen Mittels erhalten.

Weiterhin können Esterquats gemäß der Formel (Tkat1-2) verwendet werden.

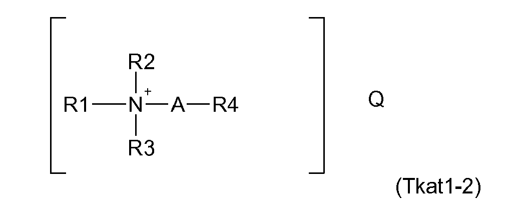

Hierin sind die Reste R1, R2 und R3 jeweils unabhängig voneinander und können gleich oder verschieden sein. Die Reste R1, R2 und R3 bedeuten:
- ein verzweigter oder unverzweigter Alkylrest mit 1 bis 4 Kohlenstoffatomen, welcher mindestens eine Hydroxylgruppe enthalten kann, beispielsweise Methyl, Ethyl, Propyl, Isopropyl, Butyl, Isobutyl, t-Butyl, Hydroxyethyl, Hydroxymethyl, oder
- ein gesättigter oder ungesättigter, verzweigter oder unverzweigter oder ein cyclischer gesättigter oder ungesättigter Alkylrest mit 6 bis 30 Kohlenstoffatomen, welcher mindestens eine Hydroxylgruppe enthalten kann, oder
- ein Aryl oder Alkarylrest, beispielsweise Phenyl oder Benzyl,
- den Rest (- A - R4), mit der Maßgabe, daß höchstens 2 der Reste R1, R2 oder R3 für diesen Rest stehen können:

Der Rest -(A - R4) ist mindestens 1 bis 3 mal enthalten.

Hierin steht A für:
1) -(CH2)n- mit n = 1 bis 20, vorzugsweise n = 1 bis 10 und besonders bevorzugt n = 1 - 5, oder
2) -(CH2-CHR5-O)n- mit n = 1 bis 200, vorzugsweise 1 bis 100, besonders bevorzugt 1 bis 50, und besonders bevorzugt 1 bis 20 mit R5 in der Bedeutung von Wasserstoff, Methyl oder Ethyl,
und R4 steht für:
1) R6-O-CO-, worin R6 einen gesättigten oder ungesättigten, verzweigten oder unverzweigten oder einen cyclischen gesättigten oder ungesättigten Alkylrest mit 6 bis 30 Kohlenstoffatomen ist, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann, oder
2) R7-CO-, worin R7 einen gesättigten oder ungesättigten, verzweigten oder unverzweigten oder einen cyclischen gesättigten oder ungesättigten Alkylrest mit 6 bis 30 Kohlenstoffatomen ist, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Rewoquat^{®}, Stepantex^{®}, Dehyquart^{®} und Armocare^{®} vertrieben. Die Produkte Armocare^{®} VGH-70, ein N,N-Bis(2-Palmitoyloxyethyl)dimethylammonium-chlorid, sowie Dehyquart^{®} F-75, Dehyquart^{®} C-4046, Dehyquart^{®} L80, Dehyquart^{®} F-30, Dehyquart^{®} AU-35, Rewoquat^{®} WE18, Rewoquat^{®} WE38 DPG und Stepantex^{®} GS 90 sind Beispiele für solche Esterquats.

Weitere erfindungsgemäß besonders bevorzugte Verbindungen der Formel (Tkat1-2) zählen zur Formel (Tkat1-2.1), den kationischen Betainestern.

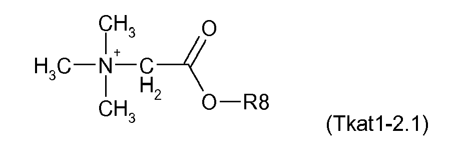

R8 entspricht in seiner Bedeutung R7.

Die Esterquats sind in den erfindungsgemäßen Zusammensetzungen in Mengen von 0,01 bis 20 Gew.%, bevorzugt in Mengen von 0,01 bis 10 Gew.% und ganz besonders bevorzugt in Mengen von 0,1 bis 7,5 Gew.% enthalten. Die allerbesten Ergebnisse werden dabei mit Mengen von 0,1 bis 5 Gew.% jeweils bezogen auf die Gesamtzusammensetzung des jeweiligen Mittels erhalten. Als weiterer Inhaltsstoff können Monoalkyltrimethylammoniumsalze mit einer Kettenlänge des Alkylrestes von 16 bis 24 Kohlenstoffatomen enthalten sein.

Diese Verbindungen weisen die in der Formel (Tkat1-1) dargestellte Struktur auf,

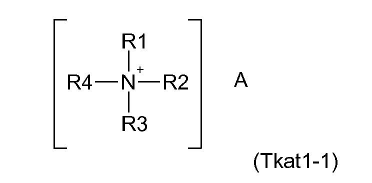

Wobei R1, R2 und R3 für jeweils eine Methylgruppe stehen und R4 für einen gesättigten, verzweigten oder unverzweigten Alkylrest mit einer Kettenlänge von 16 bis 24 Kohlenstoffatomen. Besonders bevorzugte Verbindungen der Formel (Tkat1-1) haben als Anion das Chlorid oder Methosulfat mit einer Methylgruppe als Rest R, und weiterhin als Rest R4 einen gesättigten, verzweigten oder unverzweigten Alkylrest, ganz besonders bevorzugt unverzweigten Alkylrest mit einer Kettenlänge von 18 bis 24, höchst bevorzugt mit einer Kettenlänge von 22 bis 24 Kohlenstoffatomen.

Beispiele für Verbindungen der Formel (Tkat1-1) sind Cetyltrimethylammoniumchlorid, Cetyltrimethylammoniumbromid, Cetyltrimethylammoniummethosulfat, Stearyltrimethylammoniumchlorid, Behenyltrimethylammoniumchlorid, Behenyltrimethylammoniumbromid und Behenyltrimethylammoniummethosulfat.

Die Verbindungen der Formel (Tkat1-1) werden in den erfindungsgemäßen Zusammensetzungen in einer Menge von 0,01 bis 5,0 Gew.% verwendet. Bevorzugt werden 0,1 bis 5,0 Gew.% verwendet. Besonders bevorzugt sind Mengen von 0,1 bis 3,0 Gew.%. Die Mengenangaben beziehen sich jeweils auf die gesamte Zusammensetzung.

Weiterhin enthalten die erfindungsgemäßen Mittel bevorzugt mindestens ein Amin und/oder kationisiertes Amin, insbesondere ein Amidoamin und/oder ein kationisiertes Amidoamin mit den folgenden Strukturformeln:

R¹ - NH - (CH₂)ₙ - NR²R³ (Tkat7)

und/oder

R¹ - NH - (CH₂)ₙ - NR²R³R⁴ (Tkat8)

worin R1 ein Acyl-oder Alkylrest mit 6 bis 30 C-Atomen, welche verzweigt oder unverzweigt, gesättigt oder ungesättigt sein können, und wobei der Acylrest und/oder der Alkylrest mindestens eine OH-Gruppe enthalten können, und
R2, R3 und R4 jeweils unabhängig voneinander Wasserstoff oder ein Alkylrest mit 1 bis 4 C-Atomen, welcher gleich oder verschieden, gesättigt oder ungesättigt sein kann, und
X⁻ ein Anion und
n eine ganze Zahl zwischen 1 und 10 bedeuten.

Das Anion ist ausgewählt aus den physiologisch verträglichen Anionen. Beispielhaft hierfür seien die Halogenidionen, Fluorid, Chlorid, Bromid, Sulfat der allgemeinen Formel RSO₃⁻, worin R die Bedeutung von gesättigtem oder ungesättigtem Alkylresten mit 1 bis 4 Kohlenstoffatomen hat, oder anionische Reste organischer Säuren wie Maleat, Fumarat, Oxalat, Tartrat, Citrat, Lactat oder Acetat, genannt.

Bevorzugt wird eine Zusammensetzung, in welcher das Amin und/oder das quaternisierte Amin gemäß allgemeiner Formeln (Tkat7) und/oder (Tkat8) ein Amidoamin und/oder ein quaternisiertes Amidoamin ist, worin R1 ein verzweigter oder unverzweigter, gesättigter oder ungesättigter Acylrest mit 6 bis 30 C-Atomen, welcher mindestens eine OH-Gruppe enthalten kann, bedeutet. Bevorzugt ist hierbei ein Fettsäurerest aus Ölen und Wachsen, insbesondere aus natürlichen Ölen und Wachsen, ist. Als Beispiele hierfür kommen Lanolin, Bienen-oder Candellilawachse in Betracht.

Bevorzugt sind auch solche Amidoamine und/oder quaternisierte Amidoamine, in denen R2, R3 und/oder R4 in Formeln (Tkat7) und/oder (Tkat8) ein Rest gemäß der allgemeinen Formel CH₂CH₂OR5 bedeuten, worin R5 die Bedeutung von Alkylresten mit 1 bis 4 Kohlenstoffatomen, Hydroxyethyl oder Wasserstoff haben kann. Die bevorzugte Größe von n in den allgemeinen Formeln (Tkat7) und/oder (Tkat8) ist eine ganze Zahl zwischen 2 und 5.

Weiterhin bevorzugt sind Amidoamine und/oder quaternisierte Amidoamine der allgemeinen Formeln (Tkat7) und/oder (Tkat8), in denen das Anion X⁻ ein Halogenidion oder eine Verbindung der allgemeinen Formel RSO₃⁻ ist, worin R die Bedeutung von gesättigtem oder ungesättigtem Alkylresten mit 1 bis 4 Kohlenstoffatomen hat.

Der Alkylrest mit 1 bis 4 Kohlenstoffatomen von R2, R3 und R4 und/oder der Alkylrest mit 1 bis 4 Kohlenstoffatomen von RSO₃⁻ in der allgemeinen Formel (Tkat7) und/oder (Tkat8) können mindestens eine Hydroxylgruppe enthalten.

Die Alkylamidoamine können sowohl als solche vorliegen und durch Protonierung in entsprechend saurer Lösung in eine quaternäre Verbindung in der Zusammensetzung überführt werden. Erfindungsgemäß bevorzugt sind die kationischen Alkylamidoamine.

Als erfindungsgemäß zu verwendende Amidoamine, welche gegebenenfalls quaternisiert sein können, kommen beispielsweise in Betracht als Amidoamine: Witcamine 100 (Witco, INCI-Bezeichnung: Cocamidopropyl Dimethylamine), Incromine BB (Croda, INCI-Bezeichnung: Behenamidopropyl Dimethylamine), Mackine 401 (McIntyre, INCI-Bezeichnung: Isostearylamidopropyl Dimethylamine) und andere Mackine-Typen, Adogen S18V (Witco, INCI-Bezeichnung: Stearylamidopropyl Dimethylamine), und als permanent kationische Aminoamine: Rewoquat RTM 50 (Witco Surfactants GmbH, INCI-Bezeichnung: Ricinoleamidopropyltrimonium Methosulfate), Empigen CSC (Albright&Wilson, INCI-Bezeichnung: Cocamidopropyltrimonium Chlorid), Swanol Lanoquat DES-50 (Nikko, INCI-Bezeichnung: Quatemium-33), Rewoquat UTM 50 (Witco Surfactants GmbH, Undecyleneamidopropyltrimonium Methosulfate).

Die Amidoamine oder quaternisierten Amidoamine gemäß der allgemeinen Formeln (Tkat7) und (Tkat8) können einzeln oder in beliebigen Kombinationen miteinander verwendet werden, wobei Mengen zwischen 0,01 bis 20 Gew.%, bevorzugt in Mengen von 0,01 bis 10 Gew.% und ganz besonders bevorzugt in Mengen von 0,1 bis 7,5 Gew.% enthalten. Die allerbesten Ergebnisse werden dabei mit Mengen von 0,1 bis 5 Gew.% jeweils bezogen auf die Gesamtzusammensetzung des jeweiligen Mittels erhalten.

Als besonders bevorzugter Inhaltsstoff c1) ist mindestens eine kationisch geladene polymere Verbindung enthalten. Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.

Die kationischen Polymere können Homo- oder Copolymere sein, wobei die quaternären Stickstoffgruppen entweder in der Polymerkette oder vorzugsweise als Substituent an einem oder mehreren der Monomeren enthalten sind. Die Ammoniumgruppen enthaltenden Monomere können mit nicht kationischen Monomeren copolymerisiert sein. Geeignete kationische Monomere sind ungesättigte, radikalisch polymerisierbare Verbindungen, welche mindestens eine kationische Gruppe tragen, insbesondere ammoniumsubstituierte Vinylmonomere wie zum Beispiel Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alyklvinylpyrrolidon Salze. Die Alkylgruppen dieser Monomere sind vorzugsweise niedere Alkylgruppen wie zum Beispiel C1- bis C7-Alkylgruppen, besonders bevorzugt C1- bis C3-Alkylgruppen.

Die Ammoniumgruppen enthaltenden Monomere können mit nicht kationischen Monomeren copolymerisiert sein. Geeignete Comonomere sind beispielsweise Acrylamid, Methacrylamid; Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1-bis C7-Alkylgruppen, besonders bevorzugt C1- bis C3-Alkylgruppen sind.

Geeignete Polymere mit quaternären Amingruppen sind beispielsweise die im CTFA Cosmetic Ingredient Dictionary unter den Bezeichnungen Polyquaternium beschriebenen Polymere wie Methylvinylimidazoliumchlorid/Vinylpyrrolidon Copolymer (Polyquaternium-16) oder quaternisiertes Vinylpyrrolidon/Dimethylaminoethylmethacrylat Copolymer (Polyquaternium- 11). Von den kationischen Polymeren, die in dem erfindungsgemäßen Mittel enthalten sein können, ist zum Beispiel Vinylpyrrolidon/Dimethylaminoethylmethacrylatmethosulfat Copolymer, das unter den Handelsbezeichnungen Gafquat^{®} 755 N und Gafquat^{®} 734 von der Firma Gaf Co., USA vertrieben wird und von denen das Gafquat^{®} 734 besonders bevorzugt ist, geeignet. Weitere kationische Polymere sind beispielsweise das von der Firma BASF, Deutschland unter dem Handelsnamen Luviquat^{®} HM 550 vertriebene Copolymer aus Polyvinylpyrrolidon und Imidazoliminmethochlorid, das von der Firma Calgon/USA unter dem Handelsnamen Merquat^{®} Plus 3300 vertriebene Terpolymer aus Dimethyldiallylammoniumchlorid, Natriumacrylat und Acrylamid und das von der Firma ISP unter dem Handelsnamen Gafquat^{®} HS 100 vertriebene Vinylpyrrolidon/Methacrylamidopropyltrimethylammoniumchlorid Copolymer.

Homopolymere der allgemeinen Formel (P1),

-{CH₂-[CR¹COO-(CH₂)ₘN⁺R²R³R⁴]}ₙ X⁻ (P1)

in der R¹= -H oder -CH₃ ist,
R², R³ und R⁴ unabhängig voneinander ausgewählt sind aus C1-4-Alkyl-, -Alkenyl- oder - Hydroxyalkylgruppen,
m = 1, 2, 3 oder 4,
n eine natürliche Zahl und

X⁻ ein physiologisch verträgliches organisches oder anorganisches Anion ist, sowie Copolymere, bestehend im wesentlichen aus den in Formel (Monomer-3) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R¹ steht für eine Methylgruppe
- R², R³ und R⁴ stehen für Methylgruppen
- m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Solche Produkte sind beispielsweise unter den Bezeichnungen Rheocare^{®} CTH (Cosmetic Rheologies) und Synthalen^{®} CR (3V Sigma) im Handel erhältlich.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 und Salcare^{®} SC 96 im Handel erhältlich. Copolymere enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Geeignete kationische Polymere, die von natürlichen Polymeren abgeleitet sind, sind kationische Derivate von Polysacchariden, beispielsweise kationische Derivate von Cellulose, Stärke oder Guar. Geeignet sind weiterhin Chitosan und Chitosanderivate. Kationische Polysaccharide haben die allgemeine Formel (P-3) G-O-B-N+RₐR_{b}R_{c} X⁻

G ist ein Anhydroglucoserest, beispielsweise Stärke- oder Celluloseanhydroglucose;

B ist eine divalente Verbindungsgruppe, beispielsweise Alkylen, Oxyalkylen, Polyoxyalkylen oder Hydroxyalkylen;

Rₐ, R_{b} und R_{c} sind unabhängig voneinander Alkyl, Aryl, Alkylaryl, Arylalkyl, Alkoxyalkyl oder Alkoxyaryl mit jeweils bis zu 18 C-Atomen, wobei die Gesamtzahl der C-Atome in Rₐ, R_{b} und R_{c} vorzugsweise maximal 20 ist;

X⁻ ist ein übliches Gegenanion und ist vorzugsweise Chlorid.

Eine kationische Cellulose wird unter der Bezeichnung Polymer JR^{®} 400 von Amerchol vertrieben und hat die INCI-Bezeichnung Polyquaternium-10. Eine weitere kationische Cellulose trägt die INCI-Bezeichnung Polyquaternium-24 und wird unter dem Handelsnamen Polymer LM-200 von Amerchol vertrieben. Weitere Handelsprodukte sind die Verbindungen Celquat^{®} H 100, Celquat^{®} und L 200. Die genannten Handelsprodukte sind bevorzugte kationische Cellulosen.

Geeignete kationische Guarderivate werden unter der Handelsbezeichnung Jaguar^{®} vertrieben und haben die INCI-Bezeichnung Guar Hydroxypropyltrimonium Chloride. Weiterhin werden besonders geeignete kationische Guarderivate auch von der Fa. Hercules unter der Bezeichnung N-Hance^{®} im Handel. Weitere kationische Guarderivate werden von der Fa. Cognis unter der Bezeichnung Cosmedia^{®} vertrieben. Ein bevorzugtes kationisches Guarderivat ist das Handelsprodukt AquaCat^{®} der Fa. Hercules. Bei diesem Rohstoff handelt es sich um ein bereits vorgelöstes kationisches Guarderivat.

Ein weiteres besonders geeignetes kationisches natürliches Polymer stellen Hydrokolloide von Typ der Chitosane dar. Die Chitosane oder Chitosanderivate liegen vorzugsweise in neutralisierter oder partiell neutralisierter Form vor. Ein geeignetes Chitosan wird beispielsweise von der Firma Kyowa Oil& Fat, Japan, unter dem Handelsnamen Flonac^{®} vertrieben. Ein bevorzugtes Chitosansalz ist Chitosoniumpyrrolidoncarboxylat, welches beispielsweise unter der Bezeichnung Kytamer^{®} PC von der Firma Amerchol, USA, vertrieben wird. Weitere Chitosanderivate sind unter den Handelsbezeichnungen Hydagen^{®} CMF, Hydagen^{®} HCMF und Chitolam^{®} NB/101 im Handel frei verfügbar.

Weitere bevorzugte kationische Polymere sind beispielsweise
- kationische Alkylpolyglycoside,
- kationisierter Honig, beispielsweise das Handelsprodukt Honeyquat^{®} 50,
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette,
- Vinylpyrrolidon-Vinylcaprolactam-Acrylat-Terpolymere, wie sie mit Acrylsäureestern und Acrylsäureamiden als dritter Monomerbaustein im Handel beispielsweise unter der Bezeichnung Aquaflex^{®} SF 40 angeboten werden.

Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Die im folgenden beschriebenen kationischen Proteinhydrolysate zählen nicht zu den erfindungsgemäßen kationischen Polymeren. Dennoch können sie als weitere Inhaltsstoffe in den erfindungsgemäßen Zusammensetzungen enthalten sein. Das zugrunde liegende Proteinhydrolysat kann vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen.

Ganz besonders bevorzugt sind die kationischen Proteinhydrolysate und -derivate auf pflanzlicher Basis.

Die kationischen Polymere sind in den erfindungsgemäßen Zusammensetzungen bevorzugt in Mengen von 0,01 bis 10 Gew.-%, bezogen auf das gesamte Mittel, enthalten. Mengen von 0,05 bis 5 Gew.-% sind besonders bevorzugt.

Amphotere Polymere sind ebenso wie die kationischen Polymere ganz besonders bevorzugte Polymere.

Erfindungsgemäß bevorzugte amphotere und/oder kationische Polymere sind solche Polymerisate, in denen sich eine kationische Gruppe ableitet von mindestens einem der folgenden Monomere:
- Monomeren mit quartären Ammoniumgruppen der allgemeinen Formel (Mono1),

   R¹-CH=CR²-CO-Z-(CₙH₂ₙ)-N⁽⁺⁾R³R⁴R⁵ A⁽⁻⁾ (Mono1)

   in der R¹ und R² unabhängig voneinander stehen für Wasserstoff oder eine Methylgruppe und R³, R⁴ und R⁵ unabhängig voneinander für Alkylgruppen mit 1 bis 4 KohlenstoffAtomen, Z eine NH-Gruppe oder ein Sauerstoffatom, n eine ganze Zahl von 2 bis 5 und A⁽⁻⁾ das Anion einer organischen oder anorganischen Säure ist,
- Monomeren mit quartären Ammoniumgruppen der allgemeinen Formel (Mono2), 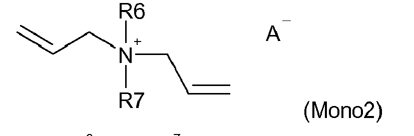 worin R⁶ und R⁷ unabhängig voneinander stehen für eine (C₁ bis C₄)-Alkylgruppe, insbesondere für eine Methylgruppe und
   A⁻ das Anion einer organischen oder anorganischen Säure ist.

Wenn sich eine kationische Gruppe der amphoteren bzw. kationischen Polymerisate vom Monomer der Formel (Mono1) ableitet, stehen die Reste R³, R⁴ und R⁵ in Formel (Mono1) bevorzugt für Methylgruppen, Z ist bevorzugt eine NH-Gruppe und A⁽⁻⁾ bedeutet bevorzugt ein Halogenid-, Methoxysulfat- oder Ethoxysulfat-Ion. Besonders bevorzugt ist es in diesem Falle Acrylamidopropyl-trimethyl-ammoniumchlorid als Monomer (Mono1) zu verwenden.

In Formel (Mono2) steht A⁻ bevorzugt für ein Halogenidion, insbesondere für Chlorid oder Bromid. Bevorzugte erfindungsgemäße amphotere Polymere sind Polymere, deren anionische Gruppe sich von mindestens einem Monomeren der Formel (Mono3) ableitet
- monomeren Carbonsäuren der allgemeinen Formel (Mono3) bzw. deren Salze mit einer organischen oder anorganischen Säure,

   R⁸-CH=CR⁹-COOH (Mono3)

   in denen R⁸ und R⁹ unabhängig voneinander Wasserstoff oder Methylgruppen sind.

Als Monomeres (Mono3) wird für die erfindungsgemäß bevorzugten amphoteren Polymerisate Acrylsäure verwendet.

Besonders bevorzugte amphotere Polymere sind Copolymere, aus mindestens einem Monomer (Mono1) bzw. (Mono2) mit dem Momomer (Mono3), insbesondere Copolymere aus den Monomeren (Mono2) und (Mono3). Erfindungsgemäß ganz besonders bevorzugt verwendete amphotere Polymere sind Copolymerisate aus Diallyl-dimethylammoniumchlorid und Acrylsäure. Diese Copolymerisate werden unter der INCI-Bezeichnung Polyquaternium-22 unter anderem mit dem Handelsnamen Merquat^{®} 280 (Nalco) vertrieben.

Darüber hinaus können die erfindungsgemäßen amphoteren Polymere neben einem Monomer (Mono1) oder (Mono2) und einem Monomer (Mono3) zusätzlich ein Monomer (Mono4)
- monomere Carbonsäureamide der allgemeinen Formel (Mono4), 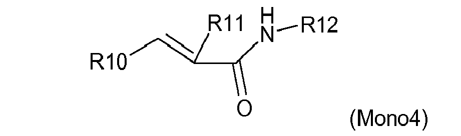 in denen R¹⁰ und R¹¹ unabhängig voneinander Wasserstoff oder Methylgruppen sind und
   R¹² für ein Wasserstoffatom oder eine (C₁- bis C₈)-Alkylgruppe steht, enthalten.

Erfindungsgemäß ganz besonders bevorzugt verwendete amphotere Polymere auf Basis eines Comonomers (Mono4) sind Terpolymere aus Diallyldimethylammoniumchlorid, Acrylamid und Acrylsäure. Diese Copolymerisate werden unter der INCI-Bezeichnung Polyquaternium-39 unter anderem mit dem Handelsnamen Merquat^{®} Plus 3330 (Nalco) vertrieben.

Die amphoteren Polymere sind in den erfindungsgemäßen Mitteln bevorzugt in Mengen von 0,01 bis 10 Gew.-%, bezogen auf das gesamte Mittel, enthalten. Mengen von 0,01 bis 5 Gew.-% sind besonders bevorzugt.

Als weiteren Inhaltstoff zusätzlich zu den zwingenden Komponenten enthalten die erfindungsgemäßen Mittel bevorzugt mindestens ein Silikonpolymer ausgewählt aus der Gruppe der Dimethiconole und/oder der Gruppe der aminofunktionellen Silikone und / oder der Gruppe der Dimethicone und / oder der Gruppe der Cyclomethicone. Diese Inhaltsstoffe werden im folgenden beschrieben.

Die erfindungsgemäßen Dimethicone können sowohl linear als auch verzweigt als auch cyclisch oder cyclisch und verzweigt sein. Lineare Dimethicone können durch die folgende Strukturformel (Si1) dargestellt werden:

(SiR¹₃) - O - (SiR²₂ - O - )ₓ - (SiR¹₃) (Si1)

Verzweigte Dimethicone können durch die Strukturformel (Si1.1) dargestellt werden:

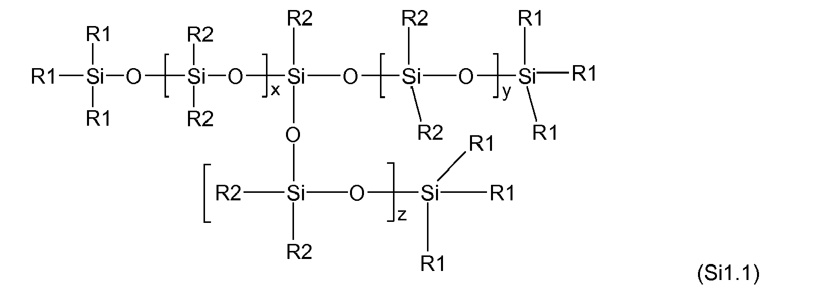

Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Die Zahlen x, y und z sind ganze Zahlen und laufen jeweils unabhängig voneinander von 0 bis 50.000. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Höchst bevorzugt sind Viskositäten um den Bereich von etwa 60.000 cPs herum. Beispielhaft sei hier auf das Produkt "Dow Corning 200 mit 60000cSt" verwiesen. Die Dimethicone (Si1) sind in den erfindungsgemäßen Zusammensetzungen in Mengen von 0,01 bis 10 Gew.%, vorzugsweise 0,01 bis 8 Gew.%, besonders bevorzugt 0,1 bis 7,5 Gew.% und insbesondere 0,1 bis 5 Gew.% bezogen auf die gesamte Zusammensetzung enthalten.
Besonders bevorzugte erfindungsgemäße Mittel enthalten ein oder mehrere aminofunktionelle Silikone.

Erfindungsgemäß geeignet sind kationische Silikonöle wie beispielsweise die im Handel erhältliche Dow Corning 929 Emulsion, DC 2-2078 (Hersteller Dow Corning, INCI-Bezeichnung: Aminopropyl Phenyl Trimethicone), DC 5-7113 (Hersteller Dow Corning, INCI-Bezeichnung: Silicone Quaternium 16), SM-2059 (Hersteller: General Electric) sowie SLM-55067 (Hersteller: Wacker).

Besonders bevorzugte erfindungsgemäße Mittel sind **dadurch gekennzeichnet, dass** sie mindestens es ein aminofunktionelles Silikon der Formel (Si3-a) 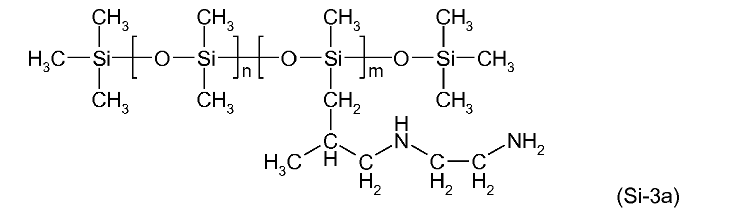 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Trimethylsilylamodimethicone bezeichnet und sind beispielsweise unter der Bezeichnung Q2-7224 (Hersteller: Dow Corning; ein stabilisiertes Trimethylsilylamodimethicon) erhältlich.

Besonders bevorzugt sind auch erfindungsgemäße Mittel, die mindestens ein aminofunktionelles Silikon der Formel (Si-3b) 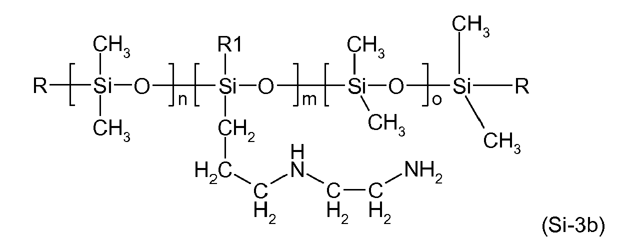 enthalten, worin
- R: für -OH, eine (gegebenenfalls ethoxylierte und/oder propoxylierte) (C₁ bis C₂₀)- Alkoxygruppe oder eine -CH₃-Gruppe steht,
- R': für -OH, eine (C₁ bis C₂₀)-Alkoxygruppe oder eine -CH₃-Gruppe und
- m, n1 und n2: Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Amodimethicone, bzw. als funktionalisierte Amodimethicone, wie beispielsweise Bis(C13-15 Alkoxy) PG Amodimethicone (beispielsweise als Handelsprodukt: DC 8500 der Firma Dow Corning erhältlich), Trideceth-9 PG-Amodimethicone (beispielsweise als Handelsprodukt Silcare Silicone SEA der Firma Clariant erhältlich) bezeichnet. Neben den zuvor beschriebenen aminofunktionellen Silikonen können bevorzugt aminofunktionelle Silikone verwendet werden, welche sich durch endständige Aminogruppen, welche wiederum besonders bevorzugt quaternär sind. Hervorragend geeignet sind hierbei diquaternäre Silikone. Geeignete diquaternäre Silikone sind ausgewählt aus Verbindungen der allgemeinen Formel (Si3c)

[R¹R ²R³N⁺ - A - SiR⁷R⁸ - (O-SiR⁹R¹⁰)ₙ - O - SiR¹¹R¹² - A - N⁺R⁴R⁵R⁶] 2X⁻ (Si3c)

wobei die Reste R1 bis R6 unabhängig voneinander C1-bis C22-Alkylreste bedeuten, welche Hydroxygruppen enthalten können und wobei vorzugsweise mindestens einer der Reste mindestens 8 C-Atome aufweist und die übrigen Reste 1 bis 4 C-Atome aufweisen,
die Reste R7 bis R12 unabhängig voneinander gleich oder verschieden sind und C1-bis C10-Alkyl oder Phenyl bedeuten,
A eine divalente organische Verbindungsgruppe bedeutet,
n eine Zahl von 0 bis 200, vorzugsweise von 10 bis 120, besonders bevorzugt von 10 bis 40 ist, und
X⁻ ein Anion ist.

Die divalente Verbindungsgruppe ist vorzugsweise eine C1-bis C12-Alkylen-oder Alkoxyalkylengruppe, die mit einer oder mehreren Hydroxylgruppen substituiert sein kann. Besonders bevorzugt ist die Gruppe -(CH₂)₃-O-CH₂-CH(OH)-CH₂-.

Das Anion X⁻ kann ein Halogenidion, ein Acetat, ein organisches Carboxylat oder eine Verbindung der allgemeinen Formel RSO₃⁻ sein, worin R die Bedeutung von C1-bis C4-Alkylresten hat.

Ein bevorzugtes diquaternäres Silikon hat die allgemeine Formel (Si3d)

[RN⁺Me₂- A - (SiMe₂O)ₙ- SiMe₂- A - N⁺Me₂R] 2 CH₃COO⁻ (Si3d),

wobei A die Gruppe -(CH₂)₃- O - CH₂ - CH(OH) - CH₂ -ist,
R ein Alkylrest mit mindestens 8 C-Atomen und n eine Zahl von 10 bis 120 ist.

Geeignete Silikonpolymere mit zwei endständigen, quaternären Ammoniumgruppen sind unter der INCI-Bezeichnung Quaternium-80 bekannt. Hierbei handelt es sich um Dimethylsiloxane mit zwei endständigen Trialkylammoniumgruppen. Derartige diquaternäre Polydimethylsiloxane werden von der Firma Evonik unter den Handelsnamen Abil^{®} Quat 3270, 3272 und 3474 vertrieben.

Erfindungsgemäß bevorzugte kosmetische Zubereitungen sind **dadurch gekennzeichnet, dass** sie, bezogen auf ihr Gewicht, 0,01 bis 10 Gew.%, vorzugsweise 0,01 bis 8 Gew.%, besonders bevorzugt 0,1 bis 7,5 Gew.% und insbesondere 0,2 bis 5 Gew.% aminofunktionelle(s) Silikon(e) und/oder diquaternäres Silikon enthalten.

Auch die nach INCI als Cyclomethicone bezeichneten cyclischen Dimethicone sind erfindungsgemäß mit Vorzug einsetzbar. Hier sind erfindungsgemäße kosmetische oder dermatologische Zubereitungen bevorzugt, die mindestens ein Silikon der Formel (Si-4) 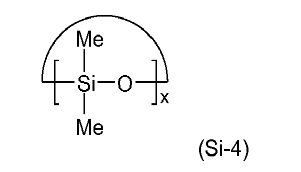 enthalten, in der x für eine Zahl von 3 bis 200, vorzugsweise von 3 bis 10, weiter bevorzugt von 3 bis 7 und insbesondere 3, 4, 5 oder 6, steht.

Erfindungsgemäß ebenfalls bevorzugte Mittel sind **dadurch gekennzeichnet, dass** sie mindestens ein Silikon der Formel (Si-5)

R₃Si-[O-SiR₂]ₓ-(CH₂)ₙ-[O-SiR₂]_{y}-O-SiR₃ (Si-5),

enthalten, in der R für gleiche oder verschiedene Reste aus der Gruppe -H, -Phenyl, -Benzyl, -CH₂-CH(CH₃)Ph, der C₁₋₂₀-Alkylreste steht, x bzw. y für eine Zahl von 0 bis 200 steht.

Als weitere Silikone neben den erfindungsgemäßen Dimethiconen, Dimethiconolen, Amodimethiconen und/oder Cyclomethiconen können wasserlösliche Silikone in den erfindungsgemäßen Zusammensetzungen enthalten sein.

Entsprechende hydrophile Silikone werden beispielsweise aus den Verbindungen der Formeln (Si-6) und/oder (Si-7) ausgewählt. Insbesondere bevorzugte wasserlösliche Tenside auf Silikonbasis sind ausgewählt aus der Gruppe der Dimethiconcopolyole die bevorzugt alkoxyliert, insbesondere polyethoxyliert oder polypropoxyliert sind.

Unter Dimethiconcopolyolen werden erfindungsgemäß bevorzugt Polyoxyalkylen-modifizierte Dimethylpolysiloxane der allgemeinen Formeln (Si-6) oder (Si-7) verstanden: 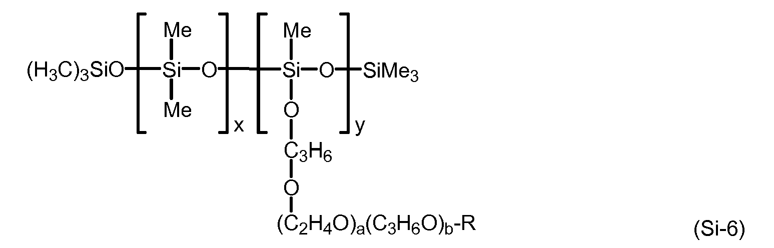 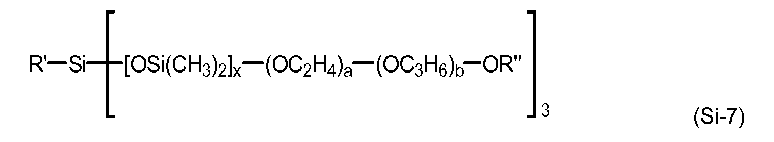 worin
- der Rest R steht für ein Wasserstoffatom, eine Alkylgruppe mit 1 bis 12 C-Atomen, eine Alkoxygruppe mit 1 bis 12 C-Atomen oder eine Hydroxylgruppe,
- die Reste R' und R" bedeuten Alkylgruppen mit 1 bis 12 C-Atomen,
- x steht für eine ganze Zahl von 1 bis 100, bevorzugt von 20 bis 30,
- y steht für eine ganze Zahl von 1 bis 20, bevorzugt von 2 bis 10 und
- a und b stehen für ganze Zahlen von 0 bis 50, bevorzugt von 10 bis 30.

Besonders bevorzugte Dimethiconcopolyole im Sinne der Erfindung sind beispielsweise die kommerziell unter dem Handelsnamen SILWET (Union Carbide Corporation) und DOW CORNING (Dow) vertriebenen Produkte.

Erfindungsgemäß besonders bevorzugte Dimethiconcopolyole sind Dow Corning 190 und Dow Corning 193 (Dow).

Die Dimethiconcopolyole sind in den erfindungsgemäßen Zusammensetzungen in Mengen von 0,01 bis 10 Gew.%, vorzugsweise 0,01 bis 8 Gew.%, besonders bevorzugt 0,1 bis 7,5 Gew.% und insbesondere 0,1 bis 5 Gew.% an Dimethiconcopolyol bezogen auf die Zusammensetzung. Schließlich werden unter den Silikonverbindungen die Dimethiconole (Si8) verstanden. Dimethiconole bilden eine weitere Gruppe der Silikone, welche erfindungsgemäß besonders bevorzugt sind. Die erfindungsgemäßen Dimethiconole können sowohl linear als auch verzweigt als auch cyclisch oder cyclisch und verzweigt sein. Lineare Dimethiconole können durch die folgende Strukturformel (Si8 - I) dargestellt werden:

(SiOHR¹₂) - O - (SiR²₂ - O - )ₓ - (SiOHR¹₂) (Si8 - I)

Verzweigte Dimethiconole können durch die Strukturformel (Si8 - II) dargestellt werden:

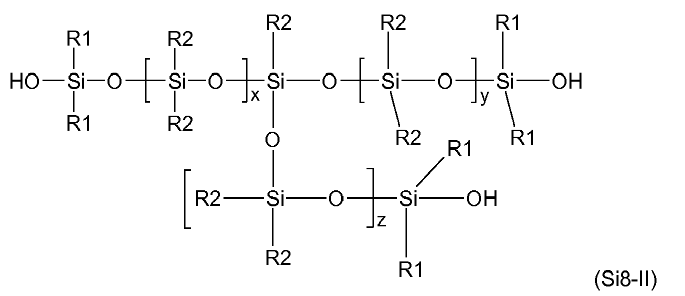

Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Die Zahlen x, y und z sind ganze Zahlen und laufen jeweils unabhängig voneinander von 0 bis 50.000. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Der bevorzugteste Bereich liegt zwischen 50000 und 2000000 cPs.

Als Beispiele für derartige Produkte werden die folgenden Handelsprodukte genannt: Dow Corning 1-1254 Fluid, Dow Corning 2-9023 Fluid, Dow Corning 2-9026 Fluid, Abil OSW 5 (Degussa Care Specialties), Dow Corning 1401 Fluid, Dow Corning 1403 Fluid, Dow Corning 1501 Fluid, Dow Corning 1784 HVF Emulsion, Dow Corning 9546 Silicone Elastomer Blend (alle zuvor genannten Dow Corning Corporation), SM555, SM2725, SM2765, SM2785 (alle zuvor genannten GE Silicones), Wacker-Belsil CM 1000, Wacker-Belsil CM 3092, Wacker-Belsil CM 5040, Wacker-Belsil DM 3096, Wacker-Belsil DM 3112 VP, Wacker-Belsil DM 8005 VP, Wacker-Belsil DM 60081 VP (alle zuvor genannten Wacker-Chemie GmbH).

Die Dimethiconole (Si8) sind in den erfindungsgemäßen Zusammensetzungen in Mengen von 0,01 bis 10 Gew.%, vorzugsweise 0,01 bis 8 Gew.%, besonders bevorzugt 0,1 bis 7,5 Gew.% und insbesondere 0,1 bis 5 Gew.% an Dimethiconol bezogen auf die Zusammensetzung.

Weiterhin können zusätzlich mit der erfindungsgemäßen Wirkstoffkombination (A) kosmetische Öle verwendet werden. Bevorzugt weisen diese Ölkörper einen Schmelzpunkt kleiner als 50 °C, besonders bevorzugt kleiner als 45 °C, ganz besonders bevorzugt kleiner als 40 °C, höchst bevorzugt kleiner als 35 °C und am bevorzugtesten sind die kosmetischen Öle bei einer Temperatur kleiner als 30 °C fließfähig. Im folgenden werden diese Öle näher definiert und beschrieben.

Zu den natürlichen und synthetischen kosmetischen Ölen sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyln-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®}OE) können bevorzugt sein.

Als natürliche Öle kommen beispielsweise Amaranthsamenöl, Aprikosenkernöl, Arganöl, Avocadoöl, Babassuöl, Baumwollsaatöl, Borretschsamenöl, Camelinaöl, Distelöl, Erdnußöl, Granatapfelkernöl, Grapefruitsamenöl, Hanföl, Haselnussöl, Holundersamenöl, Johannesbeersamenöl, Jojobaöl, Kakaobutter, Leinöl, Macadamianussöl, Maiskeimöl, Mandelöl, Marulaöl, Nachtkerzenöl, Olivenöl, Palmöl, Rapsöl, Reisöl, Sanddornfruchtfleischöl, Sanddornkernöl, Sesamöl, Sheabutter, Sojaöl, Sonnenblumenöl, Traubenkernöl, Walnußöl oder Wildrosenöl.

In vielen Fällen enthalten die Mittel mindestens eine oberflächenaktive Substanz.

Als anionische Tenside (Tanion) eignen sich in erfindungsgemäßen Zubereitungen:
- lineare und verzweigte Fettsäuren mit 8 bis 30 C-Atomen (Seifen),
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate,
- Hydroxysulfonate,
- sulfatierte Hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,
- sulfatierte Fettsäurealkylenglykolester,
- Acylglutamate,
- Kondensationsprodukte aus einem wasserlöslichen Salz eines wasserlöslichen Eiweißhydrolysates mit einer C8 - C30 Fettsäure, bevorzugt von Fettsäuren mit 12 - 18 C-Atomen. Solche Produkte sind unter dem Warenzeichen Lamepon^{®}, Maypon^{®}, Gluadin^{®}, Hostapon^{®} KCG oder Amisoft^{®} seit langem im Handel erhältlich,
- Acyllactylate,
- Hydroxymischethersulfate,
- Ethercarbonsäuren,
- Acylsarcoside,
- Acyltauride,
- Acylisethionate,
- Sulfobernsteinsäuremono- und -dialkylester,
- Alkylpolyglykolethersulfate,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate,
- Monoglyceridsulfate und Monoglyceridethersulfate,
- Amidethercarbonsäuren. Derartige Produkte sind beispielsweise von der Firma Chem-Y unter der Produktbezeichnung Akyp^{®} erhältlich.
- Acylaspartate.

Selbstverständlich können alle anionischen Tenside auch in Form ihrer Salze verwendet werden. Besonders geeignete anionische Tenside liegen jeweils in Form der Lithium-, Magnesium-, Zink-, Natrium-, Kalium- und Ammonium- sowie der Mono-, Di- und Trialkanolammoniumsalze mit 1 bis 4 C-Atomen in der Alkanolgruppe vor. Weitere anionische Tenside, welche ganz besonders bevorzugt in der erfindungsgemäßen Zusammensetzung verwendet werden, sind Alkyl- und/oder Alkenyl-Oligoglykosidcarboxylate, -sulfate, -phosphate und/oder -isethionate, die sich von Alkyl- und/oder Alkenyloligoglykosiden ableiten. Speziell bevorzugt ist ein Laurylglucosidcarboxylat, wie es als Plantapon^{®} LCG von Cognis Deutschland erhältlich ist.

Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acylaminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Typische Beispiele für amphotere bzw. zwitterionische Tenside sind Alkylbetaine, Alkylamidobetaine, Amino-propionate, Aminoglycinate, Imidazoliniumbetaine und Sulfobetaine.

Nichtionische Tenside (Tnio) enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 6 bis 30 C-Atomen, die Fettalkoholpolyglykolether bzw. die Fettalkoholpolypropylenglykolether bzw. gemischte Fettalkoholpolyether,
- Anlagerungsprodukte von 2 bis 50 Mol Ethylenoxid und/oder 0 bis 5 Mol Propylenoxid an lineare und verzweigte Fettsäuren mit 6 bis 30 C-Atomen, die Fettsäurepolyglykolether bzw. die Fettsäurepolypropylenglykolether bzw. gemischte Fettsäurepolyether,
- Anlagerungsprodukte von 2 bis 50 Mol Ethylenoxid und/oder 0 bis 5 Mol Propylenoxid an lineare und verzweigte Alkylphenole mit 8 bis 15 C-Atomen in der Alkylgruppe, die Alkylphenolpolyglykolether bzw. die Alkylpolypropylenglykolether, bzw. gemischte Alyklphenolpolyether,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol^{®} - Typen (Cognis),
- alkoxilierte Triglyceride,
- alkoxilierte Fettsäurealkylester der Formel (Tnio-1)

   R¹CO-(OCH₂CHR²)_{w}OR³ (Tnio-1)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside,
- Zuckertenside vom Typ der Fettsäure-N-alkylpolyhydroxyalkylamide,

Die kationischen Tenside (Tkat) bilden die letzte Gruppe von Tensiden und wurden bereits zuvor ausführlich beschrieben.

Kationische, zwitterionische und/oder amphotere Tenside sowie deren Mischungen können erfindungsgemäß bevorzugt sein. Anionische Tenside werden insbesondere verwendet, wenn die erfindungsgemäßen Zusammensetzungen als Duschbäder verwendet werden sollen.

Die Tenside (T) werden in Mengen von 0,05 - 45 Gew.%, bevorzugt 0,1 - 30 Gew.% und ganz besonders bevorzugt von 0,5 - 25 Gew.%, bezogen auf das gesamte erfindungsgemäß verwendete Mittel, eingesetzt.

Erfindungsgemäß verwendbare Emulgatoren sind beispielsweise
- Ethylenoxid- und Polyglycerin-Anlagerungsprodukte an Methylglucosid-Fettsäureester, Fettsäurealkanolamide und Fettsäureglucamide,
- Partialester von Polyolen mit 3-6 Kohlenstoffatomen mit gesättigten Fettsäuren mit 8 bis 22 C-Atomen,
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- sowie der unter dem Stichwort "Tenside" beschriebenen Verbindungen.

Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel.

Im folgenden werden einige Beispiele von besonders bevorzugten Polymeren beschrieben.

Bei den anionischen Polymeren handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfon-säure, das beispielsweise unter der Bezeichnung Rheothik^{®}11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylenbisacrylamid zum Einsatz kommen. Ein solches Polymer ist in dem Handelsprodukt Sepigel^{®}305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol^{®} im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze^{®} QM im Handel erhältlich. Die anionischen Polymere sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Geeignete nichtionogene Polymere sind beispielsweise:
- Vinylpyrrolidon/Vinylester-Copolymere, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) und Natrosol^{®}-Typen (Hercules) vertrieben werden.
- Stärke und deren Derivate, insbesondere Stärkeether, beispielsweise Structure^{®} XL (National Starch), eine multifunktionelle, salztolerante Stärke;
- Schellack
- Polyvinylpyrrolidone, wie sie beispielsweise unter der Bezeichnung Luviskol^{®} (BASF) vertrieben werden.

Die Polymere (P) sind in den erfindungsgemäß verwendeten Zusammensetzungen bevorzugt in Mengen von 0,01 bis 30 Gew.-%, bezogen auf die gesamte Zusammensetzung, enthalten. Mengen von 0,01 bis 25, insbesondere von 0,01 bis 15 Gew.-%, sind besonders bevorzugt. Als Fettsäuren (Fatac) können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol^{®} 871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die Einsatzmenge beträgt dabei 0,1 - 15 Gew.%, bezogen auf das gesamte Mittel. Bevorzugt beträgt die Menge 0,5 - 10 Gew.%, wobei ganz besonders vorteilhaft Mengen von 1 - 5 Gew.% sein können.

Als Fettalkohole (Fatal) können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen von bevorzugt natürlichen Fettsäuren ab. Erfindungsgemäß einsetzbar sind ebenfalls solche Fettalkoholschnitte, die ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Loro^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden. Die Fettalkohole werden in Mengen von 0,1 - 30 Gew.-%, bezogen auf die gesamte Zubereitung, bevorzugt in Mengen von 0,1 - 20 Gew.-% eingesetzt.

Als natürliche oder synthetische Wachse (Fatwax) können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Ein weiterer erfindungsgemäßer Wirkstoff in den erfindungsgemäßen Zusammensetzungen sind Proteinhydrolysate und/oder dessen Derivate (P).

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Pflanzenproteine und deren Hydrolysate sind beispielsweise Produkte auf der Basis von Weizen, Hafer, Reis, Mais, Kartoffeln, Moringa oder Soja. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda), Crotein^{®} (Croda) oder Puricare^{®} LS 9658 von der Fa. Laboratoires Sérobiologiques erhältlich.

Weitere erfindungsgemäß bevorzugte Proteinhydrolysate sind maritimen Ursprunges. Hierzu zählen beispielsweise Kollagenhydrolysate von Fischen oder Algen sowie Proteinhydrolysate von Muscheln bzw. Perlenhydrolysate. Beispiele für erfindungsgemäße Perlenextrakte sind die Handelsprodukte Pearl Protein Extract BG^{®} oder Crodarom^{®} Pearl.

Die Proteinhydrolysate (P) sind in den Zusammensetzungen in Konzentrationen von 0,001 Gew.% bis zu 20 Gew.%, vorzugsweise von 0,05 Gew.% bis zu 15 Gew.% und ganz besonders bevorzugt in Mengen von 0,05 Gew.% bis zu 5 Gew.% enthalten.

Die Wirkung der erfindungsgemäßen Zusammensetzungen kann weiterhin durch eine 2-Pyrrolidinon-5-carbonsäure und deren Derivate (J) gesteigert werden. Bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen 0,05 bis 10 Gew.%, bezogen auf das gesamte Mittel, besonders bevorzugt 0,1 bis 5, und insbesondere 0,1 bis 3 Gew.%.

Eine weitere bevorzugte Gruppe von Inhaltsstoffen der erfindungsgemäßen Zusammensetzungen mit dem erfindungsgemäßen Wirkstoffkomplex sind Vitamine, Provitamine oder Vitaminvorstufen. Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist.
- Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate. Die genannten Verbindungen des Vitamin B₅-Typs sind in den erfindungsgemäßen Mitteln bevorzugt in Mengen von 0,05 - 10 Gew.-%, bezogen auf das gesamte Mittel, enthalten. Mengen von 0,1 - 5 Gew.-% sind besonders bevorzugt.
- Vitamin B₆ (Pyridoxin sowie Pyridoxamin und Pyridoxal).

Vitamin C (Ascorbinsäure). Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S, 6aR)-2-Oxohexahydrothienol[3,4-d]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten. Schließlich ergeben sich durch die Verwendung von Pflanzenextrakten (L) in den erfindungsgemäßen Zusammensetzungen weitere Vorteile. Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Baldrian, Wiesenschaumkraut, Quendel, Schafgarbe, Thymian, Melisse, Hauhechel, Huflattich, Eibisch, Meristem, Ginseng, Kaffee, Kakao, Moringa, Ingwerwurzel, Wein, Echinacea, den Süßgräsern (Poaceaen), Litchi, insbesondere Litchi chinensis und ayurvedische Pflanzenextrakte wie Aegle Marmelos (Bilwa), Cyperus Rotundus (Nagar Motha), Emblica Officinalis (Amalki), Morida Citrifolia (Ashyuka), Tinospora Cordifolia (Guduchi), Santalum album, (Chandana), Crocus Sativus (Kumkuma), Cinnamonum Zeylanicum und Nelumbo Nucifera (Kamala) bevorzugt.

Als weiteren wesentlichen Inhaltsstoff können die erfindungsgemäßen Mittel Purin und/oder Derivat(e) des Purins enthalten. Unter Purin, den Purinen und den Purinderivaten sind erfindungsgemäß einige Vertreter besonders bevorzugt. Erfindungsgemäß bevorzugte kosmetische Mittel sind **dadurch gekennzeichnet, daß** sie Purin und/oder Purinderivat(e) der folgenden Verbindungen enthalten: Purin, Adenin, Guanin, Harnsäure, Hypoxanthin, 6-Purinthiol, 6-Thioguanin, Xanthin, Coffein, Theobromin, Theophyllin.

In haarkosmetischen Formulierungen hat sich insbesondere Coffein bewährt, das beispielsweise in Shampoos, Conditionern, Haarwässern und/oder Lotionen vorzugsweise in Mengen von 0,005 bis 0,25 Gew.-%, weiter bevorzugt von 0,01 bis 0,1 Gew.-% und insbesondere von 0,01 bis 0,05 Gew.-% (jeweils bezogen auf die Zusammensetzung) eingesetzt werden kann.

Ein weiterer bevorzugter Wirkstoff zur zusätzlichen Verwendung in den erfindungsgemäßen Mitteln ist Taurin und/oder ein Derivat des Taurines.In der vorliegenden Erfindung ist mit Taurin 2-Aminoethansulfonsäure gemeint. Bevorzugte Taurinderivate sind N-Monomethyltaurin und N,N-Dimethyltaurin. Weitere Taurinderivate im Sinne der vorliegenden Erfindung sind die Taurocholsäure und Hypotaurin.

Besonders bevorzugte sind erfindungsgemäße Mittel, die - bezogen auf ihr Gewicht - 0,0001 bis 10,0 Gew.-%, vorzugsweise 0,0005 bis 5,0 Gew.-%, besonders bevorzugt 0,001 bis 2,0 Gew.-% und insbesondere 0,001 bis 1,0 Gew.-% Taurin und/oder eines Derivates des Taurines enthalten. In den erfindungsgemäßen Mitteln sind unter geeigneten Biochinonen ein oder mehrere Ubichinon(e) und/oder Plastochinon(e) zu verstehen. Erfindungsgemäß besonders bevorzugt ist das Ubichinon der Formel mit n = 10, auch bekannt als Coenzym Q10.

Das oder die Biochinon(e) wird (werden) in den erfindungsgemäßen Mitteln - bezogen auf ihr Gewicht - in einer Menge von 0,0000005 bis 2%, bevorzugt in einer Menge von 0,000001 bis 1% und insbesondere in einer Menge von 0,00001 bis 0,5%.

Eine weitere besonders bevorzugte Gruppe von Inhaltsstoffen in den erfindungsgemäßen kosmetischen Zusammensetzungen sind Betaine.

Als Beispiele für erfindungsgemäß besonders geeignete Betaine sind zu nennen: Carnitin, Carnitintartrat, Carnitin Magnesiumcitrat, Acetylcarnitin, 3-O-Lauroyl-L-carnitin-hydrochlorid, 3-O-Octanoyl-L-carnitin-hydrochlorid, 3-O-Palmitoyl-L-carnitin-hydrochlorid, Taurinlysylat, Taurintartrat, Taurinornithat, Lysyltaurin und Ornithyltaurin, Betalaine, 1,1-Dimethyl-Prolin, Hercynin (Nα,Nα,Nα-Trimethyl-L-histidinium-betain), Ergothionein (Thionein, 2-Mercapto-Nα, Nα, Nα-trimethyl-L-histidinium-betain), Cholin, Cholinchlorid, Cholinbitartrat, Cholindihydrogencitrat und die in der Literatur als Betain bezeichnete Verbindung N,N,N-trimethylglycin. Die erfindungsgemäßen Mittel enthalten die Betaine in Mengen von 0,001 bis 20 Gew.-%, bezogen auf das gesamte Mittel. Ein Gehalt von 0,05 bis 10 Gew.-% ist bevorzugt.

Unter Polyhydroxyverbindungen sind organische Verbindungen mit mindestens zwei Hydroxygruppen zu verstehen. Insbesondere sind im Sinne der vorliegenden Erfindung hierunter zu verstehen:
- Polyole mit mindestens zwei Hydroxygruppen, wie beispielsweise Trimethylolpropan,
- Ethoxilate und/oder Propoxylate mit 1 bis 50 Mol Ethylenoxid und oder Propylenoxid der zuvor genannten Polyole,
- Kohlenhydrate, Zuckeralkohole und Zucker sowie deren Salze,
- insbesondere Monosaccharide, Disaccharide, Trisaccharide und Oligosaccharide, wobei diese auch in Form von Aldosen, Ketosen und/oder Lactosen,
- Aminodesoxyzucker, Desoxyzucker, Thiozucker, wobei diese auch in Form von Aldosen, Ketosen und/oder Lactosen.

Ganz besonders bevorzugt sind hierunter Monosaccharide mit 3 bis 8 C - Atomen, wie beispielsweise Triosen, Tetrosen, Pentosen, Hexosen, Heptosen und Octosen, wobei diese auch in Form von Aldosen, Ketosen und/oder Lactosen vorliegen können.

Weiterhin sind bevorzugt Oligosaccharide mit bis zu 50 Monomereinheiten, wobei diese auch in Form von Aldosen, Ketosen und/oder Lactosen vorliegen können.

Beispielhaft für die erfindungsgemäßen Polyole seien erwähnt Sorbit, Inosit, Mannit, Tetrite, Pentite, Hexite, Threit, Erythrit, Adonit, Arabit, Xylit, Dulcit, Erythrose, Threose, Arabinose, Ribose, Xylose, Lyxose, Glucose, Galactose, Mannose, Allose, Altrose, Gulose, Idose, Talose, Fructose, Sorbose, Psicose, Tegatose, Desoxyribose, Glucosamin, Galaktosamin, Rhamnose, Digitoxose, Thioglucose, Saccharose, Lactose, Trehalose, Maltose, Cellobiose, Melibiose, Gestiobiose, Rutinose, Raffinose sowie Cellotriose. Zusammenfassend sind erfindungsgemäße Zusammensetzungen bevorzugt, bei denen die Polyhydroxyverbindung ausgewählt ist aus Ethylenglycol, Propylenglycol, Polyethylenglycol, Polypropylenglycol, Glycerin, Glucose, Fructose, Pentaerythrit, Sorbit, Mannit, Xylit und ihren Mischungen.

Die erfindungsgemäßen Polyhydroxyverbindungen sind in den Zusammensetzungen in Konzentrationen von 0,01 Gew.% bis zu 20 Gew.%, vorzugsweise von 0,05 Gew.% bis zu 15 Gew.% und ganz besonders bevorzugt in Mengen von 0,1 Gew.% bis zu 10 Gew.% enthalten. Es kann erfindungsgemäß bevorzugt sein, das erfindungsgemäße Mittel im Rahmen einer Farbveränderung der Haare, zu verwenden. Insbesondere die oxidative Farbveränderung ist dabei bevorzugt, da die Pflegewirkung der erfindungsgemäßen Mittel auch bei Gegenwart eines Oxidationsmittels hervorragend ist.

Ferner können die kosmetischen Mittel weitere Wirk-, Hilfs- und Zusatzstoffe enthalten, wie beispielsweise
- Farbstoffe zum Anfärben des Mittels,
- Antischuppenwirkstoffe wie Piroctone Olamine, Zink Omadine und Climbazol,
- Komplexbildner wie EDTA, NTA, β-Alanindiessigsäure und Phosphonsäuren,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher verwiesen.

Wie bereits erwähnt, kommt der hohen Pflegewirkung der erfindungsgemäßen Mittel insbesondere daher Bedeutung zu, als sie auch in Gegenwart von Oxidationsmitteln - beispielsweise im Rahmen der oxidativen Haarfärbung - hervorragende Ergebnisse liefert.

Ein zweiter Erfindungsgegenstand ist daher ein Verfahren zur Haarbehandlung, in dem ein kosmetisches Mittel enthaltend
a) ein Öl aus den Früchten, insbesondere den Kernen mindestens einer Pflanze aus der Ordnung Rosales
b) mindestens einen UV - Filter,
c) mindestens eine Verbindung mit filmbildenden Eigenschaften und einen kosmetischer Träger
auf das Haar aufgetragen wird und nach einer Einwirkungszeit von wenigen Sekunden bis zu 100 Minuten vom Haar gespült wird.

Die Einwirkungszeit beträgt bevorzugt wenige Sekunden bis 100 Minuten, besonders bevorzugt 1 bis 50 Minuten und ganz besonders bevorzugt 1 bis 30 Minuten.

Die folgenden Beispiele sollen den Gegenstand der vorliegenden Erfindung erläutern ohne ihn jedoch zu beschränken.

### Beispiele

Alle Mengenangaben sind, soweit nicht anders vermerkt, Gewichtsteile.

Die folgenden Rezepturen wurden unter Anwendung bekannter Herstellungsverfahren bereitgestellt.

### Beispiel 1:

| | |
|---|---|
| Benzophenone-4 | 0,1 |
| Gafquat 755 N | 0,5 |
| Hagebuttenkernöl | 0,1 |
| Lösungsvermittler | 0,5 |
| Puffer | 0,6 |
| Konservierung | q.s. |
| Wasser, vollentsalzt | ad 100 |

### Beispiel 2:

| | |
|---|---|
| Benzophenone-4 | 0,1 |
| Panthenol | 0,2 |
| Hagebuttenkernöl | 0,1 |
| Lösungsvermittler | 0,5 |
| Puffer | 0,6 |
| Konservierung | q.s. |
| Wasser, vollentsalzt | ad 100 |

## Patentansprüche

1. Zusammensetzung zur Behandlung der Haut und von keratinischen Fasern, enthaltend
a. mindestens ein oder mehrere Öle, gewonnen aus Früchten, insbesondere den Kernen mindestens einer Pflanze aus der Ordnung Rosales,
b. mindestens ein UV - Filter,
c. mindestens eine Verbindung mit filmbildenden Eigenschaften und ein kosmetischer Träger.

2. Zusammensetzungen nach Anspruch 1, **dadurch gekennzeichnet, dass** das Öl gewonnen aus den Früchten, insbesondere den Kernen mindestens einer Pflanze aus der Ordnung Rosales ausgewählt ist aus den Ölen der Früchte, insbesondere der Kerne einer Pflanze der Familien Rosaceae, Crassulaceae oder Saxifragaceae.

3. Zusammensetzungen nach einem der Ansprüche 1 oder 2, **dadurch gekennzeichnet, dass** das Öl gewonnen aus den Früchten, insbesondere den Kernen einer Pflanze der Ordnung Rosales ausgewählt ist aus den Ölen der Früchte, insbesondere den Kernen einer Pflanze der Unterfamilie Rosoideae.

4. Zusammensetzung nach einem der Ansprüche 1 bis 3, **dadurch gekennzeichnet, dass** der UV - Filter aus mindestens einer der Gruppen der substituierten Benzophenone, p-Aminobenzoesäureester, Diphenylacrylsäureester, Zimtsäureester, Zimtsäureamidopropyl-trialkylammoniumchloride, Salicylsäureester, Octocrylene, Benzimidazole, Dodecyl-dimethylaminobenzamidopropyl-dimethylammoniumtosylate und o-Aminobenzoesäureester ausgewählt ist.

5. Zusammensetzung nach einem der Ansprüche 1 bis 4, **dadurch gekennzeichnet, dass** die Verbindung mit filmbildenden Eigenschaften ausgewählt ist aus mindestens einer der Gruppen c1), den Polymeren, insbesondere kationischen Polymeren, amphoteren Polymeren und anionischen Polymeren, der Gruppe c2), den Glycerinen und Glykolen sowie deren Ethoxilaten mit einem Ethoxilierungsgrad von 1 bis 100 Mol Ethylenoxid sowie der Gruppe c3), Vitamin B5 und dessen Derivaten.

6. Zusammensetzung nach einem der Ansprüche 1 bis 5, **dadurch gekennzeichnet, dass** die filmbildende Zusammensetzug ausgewählt ist der Gruppe c1) und der Gruppe c3).

7. Zusammensetzung nach einem der Ansprüche 1 bis 6, **dadurch gekennzeichnet, dass** weiterhin mindestens ein Esteröl enthalten ist.

8. Zusammensetzung nach einem der Ansprüche 1 bis 7, **dadurch gekennzeichnet, dass** weiterhin mindestens ein Silikon enthalten ist.

9. Verfahren zur Behandlung von Haut und keratinischen Fasern, **dadurch gekennzeichnet, dass** eine Zusammensetzung nach einem der Ansprüche 1 bis 8 auf die Haut und/oder das Haar aufgetragen, verteilt und nach einer Einwirkzeit von wenigen Sekunden bis zu 45 Minuten mit Wasser wieder ausgespült wird.

10. Verwendung einer Zusammensetzung gemäß einem der Ansprüche 1 bis 8 zur Reduzierung der Wiederanschmutzung von Haut und/oder keratinischen Fasern.

11. Verwendung einer Zusammensetzung gemäß einem der Ansprüche 1 bis 8 zur Pflege und Konditionierung von Haut und/oder keratinischen Fasern.

12. Verwendung einer Zusammensetzung gemäß einem der Ansprüche 1 bis 8 zur Restrukturierung keratinischer Fasern.

13. Verwendung einer Zusammensetzung gemäß einem der Ansprüche 1 bis 8 zur Verbesserung der Auswaschbeständigkeit gefärbter keratinischer Fasern.
